(19) Europäisches Patentamt
European Patent Office
Office européen des brevets

(11) Veröffentlichungsnummer: **0 320 796 B1**

(12) # EUROPÄISCHE PATENTSCHRIFT

(45) Veröffentlichungstag der Patentschrift: **09.03.94**

(51) Int. Cl.[5]: **C07F 9/547**, C07D 239/10, C07D 239/14, C07D 239/46, C07D 239/36

(21) Anmeldenummer: **88120519.9**

(22) Anmeldetag: **08.12.88**

Die Akte enthält technische Angaben, die nach dem Eingang der Anmeldung eingereicht wurden und die nicht in dieser Patentschrift enthalten sind.

(54) **Verfahren zur Herstellung von Phosphorsäurederivaten und Zwischenprodukten.**

(30) Priorität: **18.12.87 DE 3742983**
**14.06.88 DE 3820176**

(43) Veröffentlichungstag der Anmeldung:
**21.06.89 Patentblatt 89/25**

(45) Bekanntmachung des Hinweises auf die Patenterteilung:
**09.03.94 Patentblatt 94/10**

(84) Benannte Vertragsstaaten:
**BE CH DE ES FR GB IT LI NL**

(56) Entgegenhaltungen:
**DE-A- 3 423 623**
**FR-A- 2 365 577**
**US-A- 2 899 426**

**JOURNAL ORGANIC CHEMISTRY, vol. 31, 1966; Seiten 3838-3839***

**JOURNAL AMERICAN CHEMICAL SOCIETY, vol. 81, 1959; Seiten 2214-2219***

(73) Patentinhaber: **BAYER AG**

**D-51368 Leverkusen(DE)**

(72) Erfinder: **Maurer, Fritz, Dr.**
**Roeberstrasse 8**
**D-5600 Wuppertal 1(DE)**
Erfinder: **Lantzsch, Reinhard, Dr.**
**Am Buschhäuschen 51**
**D-5600 Wuppertal 1(DE)**
Erfinder: **Fiege, Helmut, Dr.**
**Walter Flex-Strasse 23**
**D-5090 Leverkusen 1(DE)**
Erfinder: **Schnatterer, Albert, Dr.**
**Walter Flex-Strasse 32**
**D-5090 Leverkusen 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 320 796 B1

Rank Xerox (UK) Business Services
(3.10/3.09/3.3.3)

**Beschreibung**

Die Erfindung betrifft ein neues Verfahren zur Herstellung von insektiziden Pyrimidinylphosphorsäurederivaten, Zwischenprodukte, welche für die Durchführung des Verfahrens verwendet werden können, sowie Verfahren zur Herstellung solcher Zwischenprodukte.

Es ist bereits bekannt, daß man bestimmte pestizide Phosphorsäurepyrimidinester erhält, wenn man entsprechende Phosphorsäureesterchloride mit 5-Hydroxypyrimidinen umsetzt (vgl. DE-OS 26 43 262, DE-OS 27 06 127 und DE-OS 34 23 623). US-A-2 899 426 beschreibt u. a. die Herstellung bestimmter 3,4,5,6-Tetrahydropyrimidinsalze durch die Umsetzung von Isothiouroniumsalzen mit 1,3-Diaminen. Es besteht jedoch Bedarf an neuen Verfahren zur Herstellung von gut zugänglichen Zwischenprodukten, welche in einem gut durchführbaren Herstellungsverfahren für die Synthese von Phosphorsäurepyrimidinestern eingesetzt werden können.

Es wurde nun gefunden, daß man die Verbindungen der allgemeinen Formel (I)

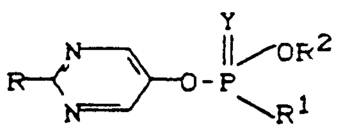

(I)

in welcher

R für Wasserstoff, für Alkoxy mit 1 bis 12 Kohlenstoffatomen, für Mono- oder Di-Alkylamino mit jeweils 1 bis 6 Kohlenstoffatomen im Alkylteil, für gegebenenfalls durch $C_1$-$C_4$-Alkoxy oder $C_1$-$C_4$-Alkylsulfonyl substituiertes Alkyl mit 1 bis 12 Kohlenstoffatomen, für gegebenenfalls durch $C_1$-$C_4$-Alkyl substituiertes Cycloalkyl mit 3 bis 8 Kohlenstoffatomen, und für gegebenenfalls durch $C_1$-$C_4$-Alkylsulfonyl substituiertes Aryl mit 6 bis 10 Kohlenstoffatomen steht,

$R^1$ für gegebenenfalls durch $C_1$-$C_4$-Alkyl, $C_1$-$C_4$-Alkoxy, $C_1$-$C_4$-Alkylthio, Halogen, Cyano und/oder Nitro substituierte Reste aus der Reihe $C_1$-$C_6$-Alkoxy, $C_1$-$C_6$-Alkylthio, Mono- oder Di-$C_1$-$C_6$-Alkylamino und Phenyl oder für gegebenenfalls durch $C_1$-$C_4$-Alkoxy, $C_1$-$C_4$-Alkylthio, Halogen, Cyano und/oder Nitro substituiertes $C_1$-$C_6$-Alkyl steht,

$R^2$ für gegebenenfalls durch $C_1$-$C_4$-Alkoxy, $C_1$-$C_4$-Alkylthio, Halogen, Cyano und/oder Nitro substituiertes $C_1$-$C_6$-Alkyl steht und

Y für Sauerstoff oder Schwefel steht,

erhält,

wenn man

a1) Verbindungen der allgemeinen Formel (IIa)

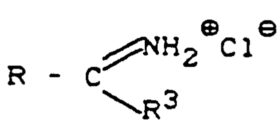

(IIa)

in welcher

R die oben angegebene Bedeutung hat,

$R^3$ für Amino oder die Gruppe $XR^4$ steht, wobei

X für Sauerstoff oder Schwefel steht und

$R^4$ für Methyl oder Ethyl steht,

mit 1,3-Diamino-2-propanol der Formel (III)

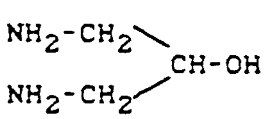

(III)

oder dessen Säureadditionssalzen,

gegebenenfalls in Gegenwart von Verdünnungsmitteln bei Temperaturen zwischen 0 °C und 120 °C

umsetzt;

a2) oder wenn man Carbonsäuren der Formel (IIb)

$R^5$-$CO_2H$ (IIb)

in welcher

$R^5$ die oben bei R angegebene Bedeutung, ausgenommen Wasserstoff, Alkoxy, Mono- und Dialkylamino, hat,

mit 1,3-Diamino-2-propanol der Formel (III)

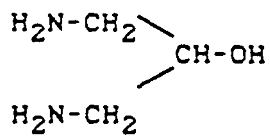
(III)

gegebenenfalls in Gegenwart von Verdünnungsmitteln bei Temperaturen zwischen 120° C und 250° C umsetzt;

oder

a3) wenn man Carbonsäuresalze des 1,3-Diamino-2-propanol der Formel (IIc)

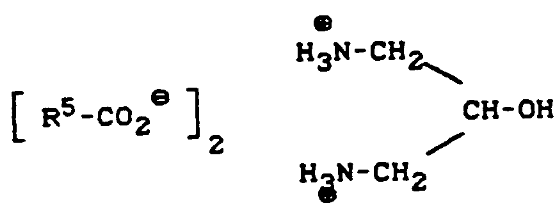
(IIc)

in welcher

$R^5$ die oben angegebene Bedeutung hat, auf Temperaturen zwischen 120° C und 250° C erhitzt, und die sich bildenden 5-Hydroxy-3,4,5,6-tetrahydropyrimidin-Salze der allgemeinen Formel (IV)

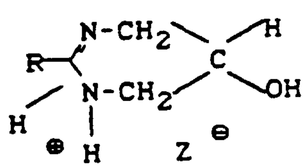
(IV)

in welcher

Z für Chlor oder $R^5$-$CO_2$ steht, wobei $R^5$ die oben angegebene Bedeutung hat und

R die oben angegebene Bedeutung hat

gegebenenfalls isoliert und

b) gegebenenfalls daraus mit einer Base die 5-Hydroxy-3,4,5,6-tetrahydropyrimidine der allgemeinen Formel (IVa)

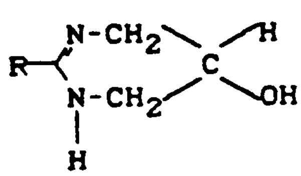
(IVa)

in welcher

R die oben angegebene Bedeutung hat,

freisetzt und anschließend

c) entweder

die 5-Hydroxy-3,4,5,6-tetrahydropyrimidin Hydrochloride der allgemeinen Formel (IV)

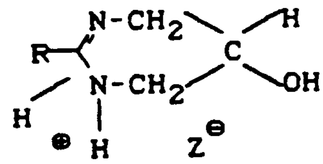

(IV)

in welcher

Z für Chlor oder $R^5$-$CO_2$ steht, wobei $R^5$ die oben angebebene Bedeutung hat und

R die oben angegebene Bedeutung hat

oder die freien 5-Hydroxy-3,4,5,6-tetrahydropyrimidine der allgemeinen Formel (IVa)

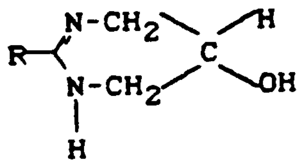

(IVa)

in welcher

R die oben angegebene Bedeutung hat,

gegebenenfalls nach ihrer Isolierung zu den Verbindungen der allgemeinen Formel (V)

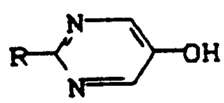

(V)

in welcher

R die oben angegebene Bedeutung hat

oxidiert bzw. dehydriert und anschließend

d) die Verbindungen der Formel (V), gegebenenfalls nach ihrer Isolierung, mit Verbindungen der allgemeinen Formel (VI)

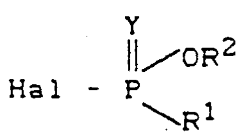

(VI)

in welcher

Hal für Halogen steht und

Y, $R^1$ und $R^2$ die oben angegebene Bedeutung haben,

gegebenenfalls in Gegenwart eines Säurebindemittels und gegebenenfalls in Gegenwart eines Lösungsmittels, umsetzt und die Verbindungen der allgemeinen Formel (I) isoliert.

Gemäß diesem Verfahren ist es möglich, die Verbindungen der Formel (I) auf einfache Weise in guter Reinheit und Ausbeute herzustellen. Das Verfahren ist im Hinblick auf die Art der gewünschten Substituenten sehr breit anwendbar. Weiterhin sind die als Zwischenprodukte einzusetzenden Verbindungen stabil und können gut gelagert und gehandhabt werden.

Bevorzugte Substituenten bzw. Bereiche der in den oben und nachfolgend erwähnten Formeln aufgeführten Reste werden im folgenden erläutert:

Alkoxy R steht für geradkettiges oder verzweigtes Alkoxy mit 1 bis 12, insbesondere 1 bis 6 und besonders

bevorzugt 1 bis 4 Kohlenstoffatomen. Beispielhaft seien Methoxy, Ethoxy, n-Propoxy, i-Propoxy, n-Butoxy, i-Butoxy, sec-Butoxy und tert-Butoxy genannt.

Mono- oder Di-Alkylamino R steht für eine Aminogruppe mit 1 oder 2 Alkylgruppen, vorzugsweise 2 Alkylgruppen, welche jeweils geradkettig oder verzweigt sein können und vorzugsweise 1 bis 5, insbesondere 1 bis 3 Kohlenstoffatome enthalten, wobei Methyl, Ethyl, n- und i-Propyl genannt seien. Beispielhaft seien Dimethylamino, Diethylamino, Di-n-propylamino und Di-i-propylamino aufgeführt.

Als gegebenenfalls substituiertes Alkyl R steht geradkettiges oder verzweigtes Alkyl mit 1 bis 12, insbesondere 1 bis 6 und besonders bevorzugt 1 bis 4 Kohlenstoffatomen. Beispielhaft seien gegebenenfalls substituiertes Methyl, Ethyl, n-Propyl, i-Propyl, n-Butyl, sec-Butyl, i-Butyl, tert-Butyl, n-Pentyl, i-Pentyl und tert-Pentyl genannt.

Als gegebenenfalls substituiertes Cycloalkyl R steht Cycloalkyl mit vorzugsweise 3 bis 8, insbesondere 3, 5 oder 6 Kohlenstoffatomen. Beispielhaft seien gegebenenfalls substituiertes Cyclopropyl, Cyclobutyl, Cyclopentyl, Cyclohexyl und Cycloheptyl genannt.

Als gegebenenfalls substituiertes Aryl R steht Aryl mit 6 bis 10 Kohlenstoffatomen im Arylteil. Beispielhaft seien gegebenenfalls substituiertes Phenyl oder Naphthyl, insbesondere Phenyl genannt.

Die in der Definition von R genannten substituierten Reste können einen oder mehrere, vorzugsweise 1 bis 3, insbesondere 1 oder 2 gleiche oder verschiedene Substituenten tragen. Als Substituenten für Alkyl, Cycloalkyl und Aryl seien beispielhaft aufgeführt:
Alkoxy und Alkylsulfonyl mit 1 bis 4 Kohlenstoffatomen, wie Methoxy, Ethoxy, n-Propoxy, i-Propoxy, n-Butoxy, i-Butoxy, sec-Butoxy, tert-Butoxy, Methylsulfonyl, Ethylsulfonyl, n-Propylsulfonyl, i-Propylsulfonyl, n-Butylsulfonyl, i-Butylsulfonyl und tert-Butylsulfonyl.

Als Arylsubstituenten und Cycloalkylsubstituenten kommt außerdem noch $C_1$-$C_4$-Alkyl in Frage, wie Methyl, Ethyl, n-Propyl, i-Propyl, n-Butyl, i-Butyl, sec-Butyl und tert-Butyl. Vorzugsweise sind die Reste R unsubstituiert.

Vorzugsweise steht

R für Wasserstoff, Alkoxy mit 1 bis 6 Kohlenstoffatomen, Mono- oder Dialkylamino mit jeweils 1 bis 4 Kohlenstoffatomen im Alkylteil oder für gegebenenfalls durch Methoxy, Ethoxy, Methylsulfonyl oder Ethylsulfonyl substituiertes Alkyl mit 1 bis 6 Kohlenstoffatomen, für gegebenenfalls durch Methyl oder Ethyl substituiertes Cycloalkyl mit 3 bis 6 Kohlenstoffatomen, und für gegebenenfalls durch Methyl, Ethyl, Methoxy, Ethoxy, Methylsulfonyl oder Ethylsulfonyl substituiertes Benzyl oder Phenyl.

Ganz besonders bevorzugt steht

R für Methyl, Isopropyl und tert-Butyl (vorzugsweise tert.-Butyl).

$R^5$ hat die gleichen allgemeinen und bevorzugten Bedeutungen wie R, wobei jedoch Wasserstoff, Alkoxy, Mono- und Dialkylamino ausgenommen sind.

Die gegebenenfalls substituierten Alkylgruppen $R^1$ und $R^2$ enthalten 1 bis 6, insbesondere 1 bis 4 und besonders bevorzugt 1 oder 2 Kohlenstoffatome.

Beispielhaft seien Methyl, Ethyl, n- und i-Propyl, n-, i-, s- und t-Butyl (vorzugsweise Ethyl und s-Butyl) genannt.

Die Alkylgruppen der gegebenenfalls substituierten Alkyl- und Dialkylaminogruppen $R^1$ haben vorzugsweise die für die Alkylgruppen $R^1$ und $R^2$ oben angegebene vorzugsweise Bedeutung. Beispielhaft seien Methyl-, Ethyl-, n- und i-Propylamino sowie Dimethyl-, Diethyl-und Methylethylamino aufgeführt.

Die Alkoxy- und Alkylthioreste $R^1$ enthalten 1 bis 6, insbesondere 1 bis 4 und besonders bevorzugt 1 oder 2 Kohlenstoffatome. Beispielhaft seien Methoxy, Ethoxy, n- und i-Propoxy sowie Methylthio, Ethylthio und n- und i-Propylthio (vorzugsweise Ethoxy) genannt.

Die gegebenenfalls substituierten Reste $R^1$ und $R^2$ können einen oder mehrere, vorzugsweise 1 bis 3, insbesondere 1 oder 2 gleiche oder verschiedene Substituenten tragen. Als Substituenten seien beispielhaft aufgeführt: Alkyl (gilt nicht für die Fälle in welchen $R^1$ bzw. $R^2$ für Alkyl steht) mit 1 bis 4, insbesondere 1 oder 2 Kohlenstoffatomen, wie Methyl, Ethyl, n- und i-Propyl, und n-, i-, s- und t-Butyl; Alkoxy mit 1 bis 4, insbesondere 1 oder 2 Kohlenstoffatomen, wie Methoxy, Ethoxy, n- und i-Propyloxy und n-, i-, s- und t-Butyloxy; Alkylthio mit 1 bis 4, insbesondere 1 oder 2 Kohlenstoffatomen, wie Methylthio, Ethylthio, n- und i-Propylthio und n-, i-, s- und t-Butylthio; Halogen, vorzugsweise Fluor, Chlor, Brom und Iod, insbesondere Chlor und Brom; Cyano und Nitro. Vorzugsweise sind die Reste $R^1$ und $R^2$ unsubstituiert.

Hal in der allgemeinen Formel (VI) steht für Fluor, Chlor, Brom und Iod, vorzugsweise für Fluor, Chlor und Brom, insbesondere für Chlor.

Y steht vorzugsweise für Schwefel und X steht vorzugsweise für Sauerstoff.

Die im Verfahrensschritt (a) als Ausgangsstoffe zu verwendenden Verbindungen sind durch die Formel (IIa), (IIb) bzw. (IIc) allgemein definiert. In dieser Formel hat R vorzugsweise die Bedeutungen, die bereits

im Zusammenhang mit der Beschreibung der Stoffe der Formel (I) für diesen Substituenten als bevorzugt genannt wurden.

Als Beispiele für die Verbindungen der Formel (IIa), in welcher $R^3$ für Amino steht, seien die folgenden Verbindungen genannt:

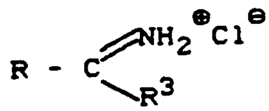

Tabelle 1

| R | R |
|---|---|
| H | $OC_2H_5$ |
| $CH_3$ | $OC_3H_7$-n |
| $C_2H_5$ | $OC_3H_7$-i |
| $C_3H_7$-n | $-CH_2OCH_3$ |
| $C_3H_7$-i | $-CH_2CH_2OCH_3$ |
| $C_4H_9$-n | $-CH_2OC_2H_5$ |
| $C_4H_9$-i | $-CH_2CH_2OC_2H_5$ |
| $C_4H_9$-s | $-CH_2SO_2CH_3$ |
| $C_4H_9$-t | $-CH_2CH_2SO_2CH_3$ |
| $C_5H_{11}$-n | $-CH_2CH_2SO_2C_2H_5$ |
| $C_5H_{11}$-t | $-N(CH_3)_2$ |
| $OCH_3$ | $-N(C_2H_5)_2$ |
| 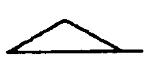 | 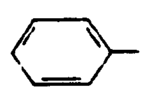 |
| 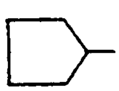 | 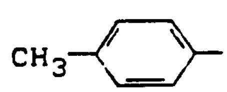 |
| 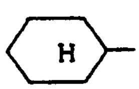 | |

Die Verbindungen der Formel (IIa) sind allgemein bekannt bzw. können nach bekannten Verfahren hergestellt werden (vgl. Org. Synthesis Coll. Vol. I, S. 5 (1951); Beilstein Bd. 2, S. 185; Bd. 2/III, S. 452; Bd. 2/III, S. 478; Bd. 9, S. 280; US-PS 4 012 506).

Die im Verfahrensschritt (a) als Ausgangsstoffe zu verwendenden Iminoalkyl(thio)ether-Hydrochloride sind durch die Formel (IIa) ebenfalls allgemein definiert. In dieser Formel steht $R^3$ für die Gruppe $XR^4$ und X und $R^4$ haben vorzugsweise die Bedeutungen, die bereits im Zusammenhang mit der Beschreibung der Stoffe der Formel (II) für X und $R^4$ als bevorzugt genannt wurden.

Als Beispiele für die Verbindungen der Formel (IIa), in welcher $R^3$ für $XR^4$ steht

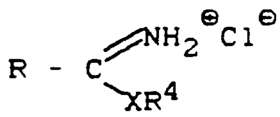

sowie für die Verbindungen der Formel (IIb)

$R^5$-$CO_2H$ (IIb)

seien genannt:

Tabelle 2

| R bzw. $R^5$ | R bzw. $R^5$ |
|---|---|
| H (zu Formel IIa) | $OC_2H_5$ |
| $CH_3$ | $OC_3H_7$-n |
| $C_2H_5$ | $OC_3H_7$-i |
| $C_3H_7$-n | $-CH_2OCH_3$ |
| $C_3H_7$-i | $-CH_2CH_2OCH_3$ |
| $C_4H_9$-n | $-CH_2OC_2H_5$ |
| $C_4H_9$-i | $-CH_2CH_2OC_2H_5$ |
| $C_4H_9$-s | $-CH_2SO_2CH_3$ |
| $C_4H_9$-t | $-CH_2CH_2SO_2CH_3$ |
| $C_5H_{11}$-n | $-CH_2CH_2SO_2C_2H_5$ |
| $C_5H_{11}$-t | $-N(CH_3)_2$ |
| $OCH_3$ | $-N(C_2H_5)_2$ |
| 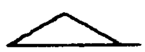 | 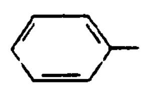 |
| 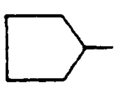 | $CH_3$ 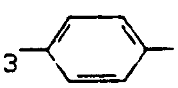 |
| 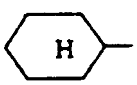 | 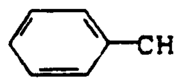 $CH_2$ |

Die Iminoalkyl(thio)ether-Hydrochloride der Formel (IIa) sind allgemein bekannte Verbindungen der organischen Chemie, bzw. können nach bekannten Verfahren hergestellt werden (vgl. Org. Synthesis Coll. Vol. I, S. 5 (1951); Beilstein Bd. 2, S. 182; Bd. 2, S. 245; Bd. 2/III, S. 451; Bd. 2/III, S. 618; Bd. 2/III, S. 675; US-PS 4 012 506).

Das im Verfahrensschritt (a) als Ausgangsstoff zu verwendende 1,3-Diamino-2-propanol der Formel (III) ist bekannt (vgl. US-PS 3 432 553). Die im Verfahrensschritt a) als Ausgangsstoffe alternativ zu verwendenden Säureadditionssalze der Formel (III) sind allgemein bekannte Verbindungen der organischen Chemie. Als Beispiele seien genannt:
1,3-Diamino-2-propanol-hydrochlorid, ˜ di-p-toluolsulfonat,
1,3-Diamino-2-propanol-dihydrochlorid, ˜ di-picrat
1,3-Diamino-2-propanol-dihydrobromid,
1,3-Diamino-2-propanol-sulfat.

Die Salze der Verbindung der Formel (III) mit den Carbonsäuren der Formel (IIb) sind nach den allgemein üblichen Salzbildungsmethoden erhältlich.

Der erfindungsgemäße Verfahrensschritt (a1) zur Herstellung der Verbindungen der allgemeinen Formel (IV) wird bevorzugt in Gegenwart von Verdünnungsmitteln durchgeführt. Als Verdünnungsmittel kommen

vorzugsweise in Frage:
Alkohole, wie Methanol, Ethanol, n- und i-Propanol, tert-Butanol, aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glykoldimethylether und Diglykoldimethylether, Tetrahydrofuran und Dioxan, Nitrile, wie z. B. Acetonitril und Propionitril, Amide, wie z. B. Dimethylacetamid und N-Methyl-pyrrolidon, sowie Tetramethylensulfon.

Die Reaktionstemperatur kann beim Verfahrensschritt (a1) innerhalb eines größeren Bereichs variiert werden. Im allgemeinen arbeitet man zwischen 0 °C und +120 °C, vorzugsweise bei +40 °C bis +80 °C. Das erfindungsgemäße Verfahren wird im allgemeinen bei Normaldruck durchgeführt.

Zur Durchführung des erfindungsgemäßen Verfahrens (a1) werden die Ausgangsstoffe gewöhnlich in äquimolaren Mengen eingesetzt. Ein Überschuß der einen oder anderen Reaktionskomponente bringt keine wesentlichen Vorteile. Die Aufarbeitung sowie die gegebenenfalls gewünschte Isolierung geschieht nach üblichen Methoden, z. B. durch Abdestillieren des Lösungsmittels unter vermindertem Druck, wobei das Produkt der Formel (IV) als Rückstand vorliegt.

Überraschenderweise kann man nach dem erfindungsgemäßen Verfahren (a1) die neuen 5-Hydroxy-3,4,5,6-tetrahydropyrimidin-Hydrochloride der allgemeinen Formel (IV) sehr leicht und in guten Ausbeuten sowie in hoher Reinheit erhalten. Sie sind daher für den Einsatz in den Verfahrensschritten (b) und (c) in besonderem Maße geeignet.

Verwendet man bei dem erfindungsgemäßen Verfahrenschritt (a1) zur Herstellung der Verbindungen der allgemeinen Formel (IV) beispielsweise Isobutyramidin-Hydrochlorid und 1,3-Diamino-2-propanol als Ausgangsstoffe, so kann die Reaktion durch das folgende Formelschema skizziert werden:

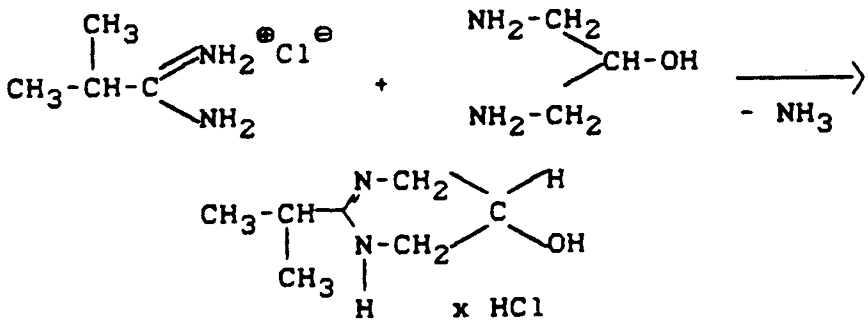


Die erfindungsgemäßen Verfahrensstufen (a2) und (a3) zur Herstellung der Verbindungen der allgemeinen Formel (IV) werden bevorzugt in Gegenwart von Verdünnungsmitteln, die ein Azeotrop mit Wasser bilden, durchgeführt. Das sich während der Reaktion bildende Wasser wird vorzugsweise laufend aus dem Reaktionsansatz entfernt.

Als Verdünnungsmittel kommen vorzugsweise in Frage:
Chlorbenzol, o-Dichlorbenzol, Xylole (ortho, meta, para, evtl. Gemische), Toluol, Methylcyclohexan, Decahydronaphthalin, Anisol, Ethylbenzol, Isopropylbenzol.

Die Reaktionstemperatur liegt im allgemeinen zwischen 120° C und 250° C. Um allzu lange Reaktionszeiten zu vermeiden, arbeitet man zweckmäßigerweise oberhalb 135° C, vorzugsweise zwischen 135° C und 200° C.

Es kann bei Normaldruck, aber auch bei erhöhtem oder vermindertem Druck gearbeitet werden.

Bei der Durchführung des Verfahrensschrittes (a2) wird auf ein Mol Diaminopropanol der Formel (III) oder dessen Carbonsäuresalz 1,5 bis 3, vorzugsweise 2 bis 2,5 Mol der Carbonsäure der Formel (IIb) eingesetzt.

Die Aufarbeitung und Isolierung der Verbindungen der Formel (IV) erfolgt durch Abfiltrieren oder Abtrennen der sich bildenden Phase, die die Verbindungen der Formel (IV) enthält.

Verwendet man bei dem erfindungsgemäßen Verfahrensschritt (a2) beispielsweise Isobuttersäure und 1,3-Diamino-2-propanol als Ausgangsstoffe, so kann die erste Stufe der Reaktion durch das folgende Formelschema skizziert werden:

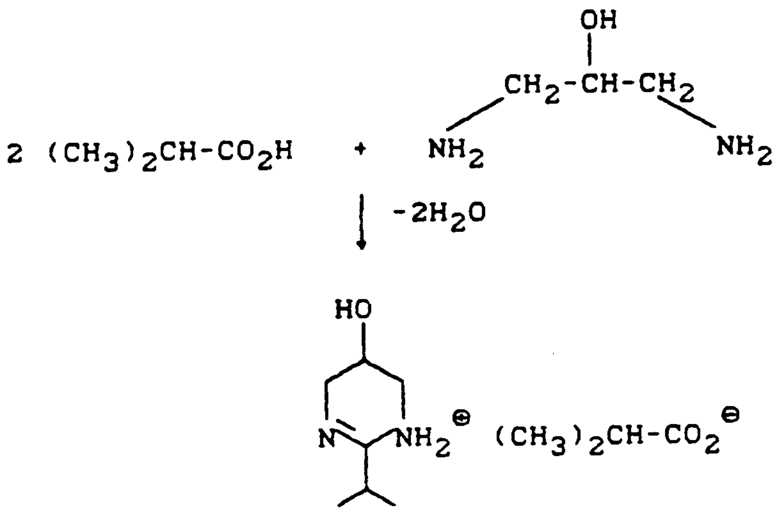


Verwendet man bei dem erfindungsgemäßen Verfahrensschritt (a3) beispielsweise das Di-Pivalinsäuresalz des 1,3-Diamino-2-propanol als Ausgangsstoff, so kann die Reaktion durch das folgende Formelschema skizziert werden:

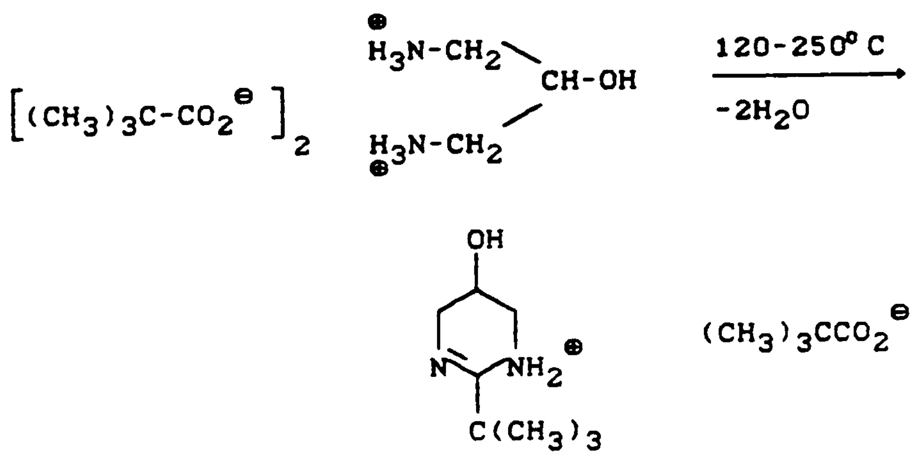


Überraschenderweise kann man nach dem erfindungsgemäßen Verfahrensschritt (a2) die 5-Hydroxy-3,4,5,6-tetrahydropyrimidin-Derivate der Formel (IV) in guten Ausbeuten und hoher Reinheit erhalten.

Insbesondere war es durch den Fachmann nicht zu erwarten, daß die Carbonsäuren der Formel (IIb) bereits bei relativ niedriger Temperatur reagieren, zumal aus der Literatur bekannt war, daß eine analoge Reaktion mit Diaminopropan Temperaturen von 225° C erfordert (EP-PS 117 882).

Darüber hinaus wird, um eine Monoacylierung zu erhalten, in der genannten Patentanmeldung Diaminopropan vorzugsweise in großem Überschuß eingesetzt. Es ist somit als äußerst überraschend anzusehen, daß trotz überschüssigem Einsatz der Carbonsäure der Formel (IIb) die Reaktion in so guter Ausbeute verläuft.

Das erfindungsgemäße Verfahren ist ferner als ausgesprochen überraschend zu bezeichnen, weil der Fachmann unter den sauren Reaktionsbedingungen mit einer Dehydratisierung der sekundären Alkoholgruppe oder mit einer Veresterung der Alkoholgruppe mit den Carbonsäuren der Formel (IIb) rechnen mußte.

Bei der Verfahrensvarianten (a3) werden die Verfahrensbedingungen (Lösungsmittel, Temperaturen, Aufarbeitung und Isolierung) wie beim Verfahren (IIb) dargestellt, durchgeführt.

Als Beispiele für die erfindungsgemäß erhältlichen Verbindungen der Formel (IV) seien die folgenden Verbindungen aufgeführt:

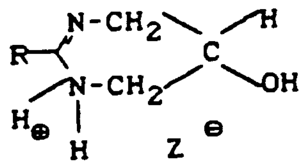 (IV)

Z = Cl, $R^5CO_2$

<u>Tabelle 3</u>

| R bzw. $R^5$ | R bzw. $R^5$ |
|---|---|
| H (Z = Cl) | $OC_2H_5$ |
| $CH_3$ | $OC_3H_7$-n |
| $C_2H_5$ | $OC_3H_7$-i |
| $C_3H_7$-n | $-CH_2OCH_3$ |
| $C_3H_7$-i | $-CH_2CH_2OCH_3$ |
| $C_4H_9$-n | $-CH_2OC_2H_5$ |
| $C_4H_9$-i | $-CH_2CH_2OC_2H_5$ |
| $C_4H_9$-s | $-CH_2SO_2CH_3$ |
| $C_4H_9$-t | $-CH_2CH_2SO_2CH_3$ |
| $C_5H_{11}$-n | $-CH_2CH_2SO_2C_2H_5$ |
| $C_5H_{11}$-t | $-N(CH_3)_2$ |
| $OCH_3$ | $-N(C_2H_5)_2$ |
| 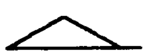 | 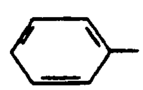 |
| 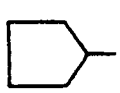 | 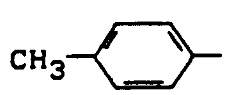 |
| 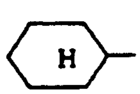 | 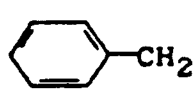 |

Das Verfahren zur Herstellung der Verbindungen der allgemeinen Formel (IV) gemäß Verfahrensschritt (a1), (a2) und (a3) ist noch nicht aus der Literatur bekannt und Teil der vorliegenden Erfindung.

Diese Verbindungen können beispielsweise in der Verfahrensstufe (c) eingesetzt werden.

Die ebenfalls in der Verfahrensstufe (c) einzusetzenden Verbindungen der allgemeinen Formel (IVa) sind teilweise bekannt. So ist es bereits bekannt, daß 1,3-Diamino-propan-2-ol mit Ameisensäureethylester zu 5-Hydroxy-3,4,5,6-tetrahydropyrimidin kondensiert und mit Essigsäureethylester 5-Hydroxy-2-methyl-3,4,5,6-tetrahydropyrimidin ergibt (vgl. J. Org. Chem., 3838 -3839, (1966)). Die Nachteile dieses Verfahrens bestehen in den mäßigen Ausbeuten und der aufwendigen Aufarbeitung der Endprodukte.

Es wurde gefunden, daß man die 5-Hydroxy-3,4,5,6-tetrahydropyrimidine der allgemeinen Formel (IVa)

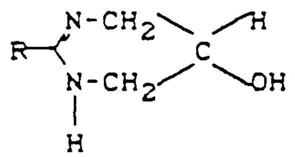

(IVa)

in welcher

R die oben angegebene Bedeutung hat

erhält, wenn man 5-Hydroxy-3,4,5,6-tetrahydropyrimidin-Hydrochloride der allgemeinen Formel (IV)

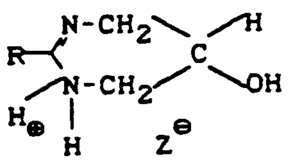

(IV)

in welcher

R und Z die oben angegebenen Bedeutungen haben,

mit einer Base, vorzugsweise mit einer wäßrigen Lösung von Alkalihydroxid, insbesondere von Natriumhydroxid und/oder Kaliumhydroxid umsetzt.

Es ist als überraschend zu bezeichnen, daß man nach dem erfindungsgemäßen Verfahrensschritt (b) die neuen 5-Hydroxy-3,4,5,6-tetrahydropyrimidine in guten Ausbeuten und in hoher Reinheit auf technisch einfachem Wege erhält, da bekannt war, daß 3,4,5,6-Tetrahydro-5-hydroxypyrimidin und 3,4,5,6-Tetrahydro-2-methyl-5-hydroxypyrimidin sehr leicht hydrolisieren.

Verwendet man bei dem erfindungsgemäßen Verfahrensschritt (b) beispielsweise 3,4,5,6-Tetrahydro-2-isopropyl-5-pyrimidinol-Hydrochlorid als Ausgangsstoff und Natriumhydroxid als Base, so kann die Reaktion durch das folgende Formelschema skizziert werden:

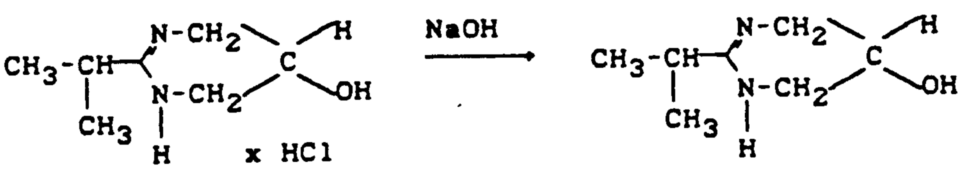


Als Beispiele für die erfindungsgemäß erhältlichen Verbindungen der Formel (IVa) seien die freien Basen der in Tabelle 3 angegebenen Verbindungen der Formel IV aufgeführt.

Bei der Durchführung des erfindungsgemäßen Verfahrens zur Herstellung der Verbindungen der allgemeinen Formel (IVa) wird vorzugsweise der Ausgangstoff der Formel (IV) in Wasser vorgelegt und die wäßrige Alkalilauge (vorzugsweise Natronlauge oder Kalilauge) zugetropft.

Bei der Durchführung des erfindungsgemäßen Verfahrensschrittes (b) setzt man ein Mol Ausgangsstoff der Formel (IV) mit 1 bis 2 Mol, vorzugsweise 1 bis 1,5 Mol Alkalilauge um.

Die Reaktion wird bei Raumtemperatur oder leicht erhöhten Temperaturen durchgeführt.

Im allgemeinen arbeitet man zwischen 10 °C und 50 °C, vorzugsweise jedoch zwishen 10 °C und 30 °C. Insbesondere wird bei Raumtemperatur umgesetzt.

Die Konzentration der wäßrigen Alkalihydroxidlösung kann zwischen 10 % und 50 % liegen, vorzugsweise jedoch zwischen 20 % und 45 %. (Bei den Prozentangaben handelt es sich um Gewichtsprozente.)

Die Aufarbeitung und gegenenfalls gewünschte Isolierung der Verbindungen der allgemeinen Formel (IVa) geschieht nach üblichen Methoden, wie beispielsweise durch Absaugen.

Die Verbindungen der allgemeinen Formel (IVa) können aus den Salzen der Formel (IV) auch mit Hilfe von stark basischen Ionenaustauschern erhalten werden. Hierzu können übliche (im Handel erhältliche)

Ionenaustauscher verwendet werden. Die Umsetzung erfolgt nach üblichen Methoden. Vorzugsweise werden wäßrige Lösungen der Verbindungen der Formel (IV) mit einer ausreichenden Menge des Ionenaustauschers gerührt, der Ionenaustauscher abfiltriert und das Wasser von Filtrat entfernt (z.B. durch möglichst schonendes Abdestillieren oder bei sehr empfindlichen Basen durch Gefriertrocknung).

Die in der Verfahrensstufe (d) einzusetzenden Verbindungen der allgemeinen Formel (V) sind bekannt bzw. können nach allgemein bekannten Methoden hergestellt werden.

So ist es bereits bekannt, daß man 5-Hydroxy-pyrimidine erhält, wenn man 5-Methoxy-pyrimidine im Autoklaven, bei Temperaturen zwischen 180 °C und 200 °C unter basischen Bedingungen umsetzt (vgl. z. B. DE-OS 26 43 262 und Coll. Czech. Chem. Comm. 40, 1078 ff (1975)). Die Nachteile dieser Verfahren bestehen darin, daß die Ausbeuten und die Reinheit der Reaktionsprodukte häufig unbefriedigend sind und außerdem extreme Reaktionsbedingungen erforderlich sind.

Es ist außerdem bekannt, daß man die 5-Hydroxy-pryimidine auch in Gegenwart von Alkalihydroxiden und Glykol aus 5-Methoxy-pyrimidinen herstellen kann. Bei diesem Verfahren sind Temperaturen von ca. 200 °C erforderlich. Weitere Nachteile sind die aufwendige Aufarbeitung der Endprodukte und die mäßigen Ausbeuten (vgl. z. B. J. Chem. Soc. 1960, 4590 ff und Chem. Ber. 95, 803 ff (1962)). Außerdem verlangt die Arbeitsweise in polaren hochsiedenden Lösungsmitteln wie Glykol besondere Anstrengungen zur Abwasserreinigung.

Weiterhin ist bekannt, daß man 5-Hydroxy-pyrimidine auch mit Wasserstoff in Gegenwart von Hydrierungskatalysatoren aus 4-Chlor-pyrimidin-Derivaten herstellen kann. Der Nachteil dieses Verfahrens liegt in der aufwendigen Herstellung der 4-Chlorpyrimidine (vgl. DE-OS 34 23 623).

Es wurde gefunden, daß man die 5-Hydroxy-pyrimidine der allgemeinen Formel (V)

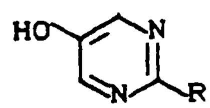

(V)

in welcher

R die oben angegebene Bedeutung hat,

erhält, wenn man entweder substituierte 5-Hydroxy-3,4,5,6-tetrahydropyrimidin-Salze der allgemeinen Formel (IV)

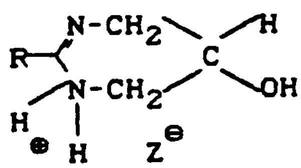

(IV)

in welcher

R und Z die oben angegebenen Bedeutungen haben

oder

5-Hydroxy-3,4,5,6-tetrahydropyrimidine der allgemeinen Formel (IVa)

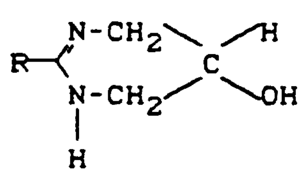

(IVa)

in welcher

R die oben angegebenen Bedeutung hat,

gegebenenfalls nach ihrer Isolierung, mit Oxidationsmitteln, gegebenenfalls in Gegenwart von Dehydrierungskatalysatoren und gegebenenfalls in Gegenwart von Verdünnungsmitteln bei Temperaturen zwischen 5

° C und 150 ° C umsetzt.

Überraschenderweise ist es möglich mit Hilfe dieses Verfahrens, welches dem Verfahrensschritt (c) entspricht und welches Teil der vorliegenden Erfindung ist, unter relativ milden Bedingungen die 5-Hydroxypyrimidine der allgemeinen Formel (V) in sehr hoher Reinheit zu erhalten. Weitere Vorteile des Verfahrens sind die Rückgewinnung der Katalysatoren und die Verwendung von preisgünstigen und umweltfreundlicheren Verdünnungsmitteln.

Verwendet man beispielsweise für das erfindungsgemäße Verfahren 3,4,5,6-Tetrahydro-2-tert-butyl-5-hydroxypyrimidin als Ausgangsstoff und Sauerstoff als Oxidationsmittel, so kann die Reaktion durch das folgende Formelschema skizziert werden:

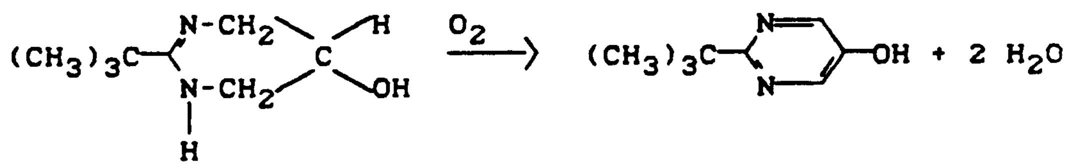

Für die Herstellung der Verbindungen der allgemeinen Formel (V) aus den Verbindungen der allgemeinen Formel (IV) und (IVa) wird vorzugsweise Wasser als Lösungsmittel verwendet.

Die Oxidationsmitel bzw. Dehydrierungsmittel für das erfindungsgemäße Verfahren sind an sich bekannt (Organikum, VEB Deutscher Verlag der Wissenschaften, Berlin 1977, Seiten 430-471). Beispielsweise seien Salpetersäure, Sauerstoff und seine Perverbindungen (Wasserstoffperoxid, Metallperoxid, anorganische und organische Persäuren), Schwefel, Selendioxid, Chlor, Brom, unterhalogenige Säuren, Chlorsäure, Periodsäure, Metallverbindungen höherer Wertigkeitsstufen [Eisen-(II)-verbindungen, Mangandioxid, Kaliumpermanganat, Chromsäure, -anhydrid, Bleidioxid und Bleitetraacetat], Tetrachlor-p-benzochinon und Dichlordicyanbenzochinon (DDQ) genannt. Bevorzugte Oxidationsmittel sind Kalium-permanganat, Ammonium-, Natrium- oder Kalium-peroxodisulfat und Chromtrioxid.

Es kann auch vorteilhaft sein, das erfindungsgemäße Verfahren in Gegenwart von Dehydrierungskatalysatoren durchzuführen. Es werden vorzugsweise Metallkatalysatoren der VIII. Nebengruppe des Periodensystems, wie beispielsweise Platin oder Paladium, gegebenenfalls auf üblichen Trägermaterialien, wie beispielsweise Aktivkohle, Siliciumdioxid oder Aluminiumoxid, eingesetzt.

Die Reaktionstemperaturen können bei der Durchführung des erfindungsgemäßen Verfahrens in einem größeren Bereich variiert werden. Im allgemeinen arbeitet man bei Temperaturen zwischen +10° C und +180° C, vorzugsweise bei Temperaturen zwischen + 20° C und +150° C, insbesondere zwischen + 20° C und +100° C.

Das erfindungsgemäße Verfahren wird im allgemeinen bei Normaldruck durchgeführt. Unter bestimmten Voraussetzungen, insbesondere bei der Verwendung von Dehydrierungskatalysatoren, kann es jedoch vorteilhaft sein, auch unter erhöhtem Druck zu arbeiten.

Zur Durchführung des erfindungsgemäßen Verfahrens setzt man im allgemeinen auf 1 Mol 5-Hydroxy-3,4,5,6-tetrahydropyrimidin bzw. Hydrochlorid der Formel (IV) oder (IVa) zwischen 0,5 und 5 Mol, vorzusweise zwischen 0,6 und 4 Mol Oxidationsmittel und/oder zwischen 0,01 und 1 Mol, vorzugsweise zwischen 0,05 und 0,5 Mol Dehydrierungskatalysator ein.

In einer besonders bevorzugten Ausführungsform wird die Oxidation (Dehydrierung) der Verbindungen der allgemeinen Formel (IV) und (IVa) zu den Verbindungen der allgemeinen Formel (V) mit Sauerstoff als Oxidationsmittel durchgeführt. In einer ganz besonders bevorzugten Ausführungsvariante wird die Dehydrierung mit Sauerstoff (Luft) in flüssiger Phase in Gegenwart eines Lösungsmittels und einer Base durchgeführt. Als vorteilhaft erweist sich der Zusatz eines Schwermetallsalzes (vorzugsweise einer Übergangsmetallverbindung).

Durch diese Verfahrensvariante des Verfahrensschritts (c) ist es überraschenderweise möglich, die 5-Hydroxypyrimidine in besonders hoher Ausbeute und hoher Reinheit herzustellen. Dieser Verfahrensschritt zeichnet sich weiterhin durch die leichte Verfügbarkeit der Ausgangskomponenten, durch ein billiges und leicht zu handhabendes Oxidationsmittel, durch milde Reaktionsbedingungen und durch eine sehr einfache Aufarbeitung aus.

Lösungsmittel für das erfindungsgemäße Verfahren zur Herstellung von (V) durch Dehydrierung von (IV) oder (IVa) mit Sauerstoff im Verfahrensschritt (c) sind solche, die mit Sauerstoff nicht oder nur vergleichsweise langsam reagieren und die Ausgangsmaterialien wenigstens teilweise lösen. Als Beispiele seien genannt: Alkohole, Etheralkohole, Kohlenwasserstoffe, chlorierte Kohlenwasserstoffe, Amine, Nitrile, Amide, Ether, Polyether, Ester, Sulfoxide, Sulfone, Lactone, Lactame usw. Aus diesen werden bevorzugt Alkohole,

Etheralkohole, Amine, Sulfoxide, Sulfone, Amide, Ether und Polyether, insbesondere Alkohole, Amine, Amide, Sulfoxide und Sulfone eingesetzt. Einzelbeispiele aus dem Bereich Alkohole und Etheralkohole sind: Methanol, Ethanol, n- und i-Propanol, 1-Butanol, 2-butanol, tert.-Butonal, Isopentylalkohol, Isohexanol, Isooctanol, Diethylenglykol, Tetraethylenglykol, usw. Aus dem Bereich der Amine seien beispielhaft aufgeführt: Triethylamin, Dibutylamin, Ethylendiamin, Tetramethylethylendiamin, Permethyldiethylentriamin, Pyridin, Picolin, Chinolin, Dimethylanilin, Diphenylamin, Di- und Tribenzylamin. Einzelbeispiele aus dem Bereich Sulfoxide und Sulfone sind Dimethylsulfoxid, Methylphenylsulfoxid, Dimethylsulfon usw. Einzelbeispiele aus dem Bereich Amide sind Formamid, Acetamid, Dimethylformamid, Dimethylacetamid, N-Methylpyrrolidon usw. Einzelbeispiele aus dem Bereich Ether und Polyether sind: Methyl-tert.-butylether, Di-tert.-butylether, Diethylenglykoldimethylether, Triethylenglykoldimethylether usw. Besonders bevorzugt sind die Lösungsmittel t-Butanol, Dimethylsulfoxid und die genannten Amine und Amide, insbesondere Dimethylformamid, N-Methylpyrrolidon, Pyridin und Picolin, sowie Mischungen dieser Lösungsmittel.

Als Basen für die Dehydrierung mit Sauerstoff eignet sich eine Vielfalt der bekannten Basen wie Amine, Metallcarbonate, Metallhydroxide, Metallalkoholate, Metallamide, quartäres Ammoniumhydroxid usw. Die Wahl der Base richtet sich insbesondere nach der Wahl der Reaktionsbedingungen wie Lösungsmittel und Reaktionstemperatur.

Bevorzugt eingesetzt werden die Hydroxide, Alkoholate und Amide der Alkali- und Erdalkalimetalle und des Aluminiums. Besonders bevorzugte Alkali- und Erdalkalimetalle sind hierbei Natrium, Kalium, Lithium, Magnesium, Calcium, Barium. Als Alkoholate seien beispielhaft das Methylat, Ethylat, Isopropylat, sek. Butylat, tert.-Butylat und das Salz des Tetraethylenglykols genannt. Die Amide können beispielsweise unsubstituiertes Amid, Ethylamid, Diethylamid, Diisopropylamid, Dibutylamid usw. sein. Unter den genannten Basen sind Hydroxide und Alkoholate bevorzugt, insbesondere bevorzugt sind Natriumhydroxid, Kaliumhydroxid, Natriummethylat, Natriumethylat und Kalium-tert.-butylat. Bevorzugte Basen sind ebenfalls quartäres Ammoniumhydroxid z.B. Tetramethylammoniumhydroxid und stark basische Ionenaustauscher z.B. solche mit quartärem Ammoniumhydroxid als funktioneller Gruppe. Die Base kann gegebenenfalls auch in Kombination mit einem Kronenether z.B. 18-Crown-6 eingesetzt werden.

Die Base wird in einer Menge von 1-15 Äquivalenten pro Mol des 5-Hydroxy-tetrahydropyrimidins (IV) oder (IVa), bevorzugt 1-10 Äquivalenten, besonders bevorzugt 2-7 Äquivalenten eingesetzt.

Vorteilhaft, aber nicht zwingend notwendig, werden dem Reaktionsgemisch im erfindungsgemäßen Verfahren zur Herstellung von Verbindungen der Formel (V) durch Oxidation von Verbindungen der Formel (IV) oder Verbindungen der Formel (IVa) mit Sauerstoff im Verfahrensschritt (c) Schwermetallsalze, vorzugsweise Übergangsmetallverbindungen, als Katalysatoren zugesetzt. Als wirksam erweisen sich diejenigen Übergangsmetalle, welche bekanntermaßen Oxidation, Autoxidationen und Dehydrierungen katalysieren, z.B. Vanadium, Chrom, Mangan, Eisen, Kobalt, Nickel, Kupfer, Silber, Niob, Ruthenium, Molybdän, Palladium, Wolfram, Platin. Unter diesen sind Kupfer, Mangan, Kobalt, Nickel, Eisen und Ruthenium bevorzugt, im besonderen bevorzugt werden Kupfer, Kobalt und Mangan und ganz besonders bevorzugt ist das Kupfer. Diese Metalle können einzeln oder in beliebigen Kombinationen und in jeder der individuell möglichen Oxidationsstufe zugesetzt werden.

Mögliche Einsatzformen solcher Metallverbindungen sind die Metallsalze anorganischer Säuren, beispielsweise die Halogenide wie Fluoride, Chloride, Bromide oder Jodide, die Sulfate, Nitrate, Carbonate, Phosphate, Borate, Sulfite, Cyanide oder die Salze organischer Säuren, wie die Acetate, Stearate, Oxalate oder Ionenaustauscher, die solche Metalle gebunden enthalten. Es können weiterhin auch Metallkomplexe bzw. Komplexsalze, z.B. Aminkomplexe, Halogenkomplexe und Metallchelatkomplexe z.B. Metallacetylacetonate, Metallglyoximate, Metallphthalocyanine, Metallporphyrine oder Metallkomplexe mit dem Liganden Bis-salicylaldehyd-ethylendiamin eingesetzt werden. Die Metalle können aber auch in elementarer Form dem Reaktionsgemisch zugesetzt werden.

In bevorzugter Weise kommen die Metallverbindungen in Form der Salze anorganischer oder organischer Säuren (besonders bevorzugt anorganischer Säuren) zur Anwendung, insbesondere als Chloride, Sulfate, Nitrate, Oxide, Hydroxide, Acetate usw. in hydratisierter oder dehydratisierter Form. In besonders bevorzugter Weise werden anorganische und organische Salze von Kupfer eingesetzt z.B. CuO, $CuCl_2 \cdot 2 H_2O$, $CuSO_4$, $Cu(OAc)_2$, CuCl usw.

Die Einsatzmenge des Metallkatalysators ist keiner spezifischen Beschränkung unterworfen. Jede beliebige Menge, soweit sie 0,0001 Moläquivalente, bezogen auf die Verbindungen der Formel (IV) bzw. (IVa) nicht unterschreitet, ist wirksam. Bevorzugt ist der Bereich von 0,0005-0,10, besonders bevorzugt ist der Bereich von 0,001-0,05 Moläquivalenten, bezogen auf die Verbindungen der Formel (IV) bzw. (IVa).

Als Sauerstoff für das erfindungsgemäße Verfahren zur Herstellung von Verbindungen der Formel (V) durch Sauerstoffoxidation von Verbindungen der Formel (IV) oder (IVa) im Verfahrensschritt (c) kann reiner Sauerstoff oder Sauerstoff in verdünnter Formel, beispielsweise in Form von Sauerstoff enthaltenden Gasen,

vorzugsweise Luft oder Sauerstoff-Stickstoff-Gemische eingesetzt werden. Die wirtschaftlich günstigste Form des erfindungsgemäß verwendbaren Sauerstoffs ist die atmosphärische Luft. Der Druck des Sauerstoff bzw. des Sauerstoff enthaltenden Gases ist keiner besonderen Einschränkung unterworfen und kann zwischen 1-100 bar liegen, vorzugsweise bei 1-10 bar. Der Sauerstoffgehalt ist bei Verwendung von Sauerstoff enthaltenden Gasen ebenfalls keiner Beschränkung unterworfen. Er richtet sich vorzugsweise nach betrieblichen Gesichtspunkten, wie der Betriebssicherheit und der Umsetzungsgeschwindigkeit. Die Zufuhr des Sauerstoffs zum Reaktionsmedium kann beispielsweise unter Verwendung von Fritten erfolgen; er kann jedoch auch durch kräftiges Rühren in das Reaktionsgemisch eingezogen werden.

Die Reaktionstemperatur kann in weiten Grenzen schwanken und liegt vorzugsweise bei 0-300° C, besonders bevorzugt bei 20-200° C und ganz besonders bevorzugt bei 40 bis 120° C.

Die Oxidation der Verbindungen der Formel (IV) oder (IVa) zu Verbindungen der Formel (V) mit Sauerstoff nach dem erfindungsgemäßen Verfahren wird vorzugsweise in flüssiger Phase durchgeführt. Die flüssigen und festen Ausgangskomponenten können entweder zu Beginn der Reaktion vollständig zusammengegeben werden, oder aber eine oder mehrere Komponenten, z.B. die Base und/oder die Verbindungen der Formeln (IV) bzw. (IVa) werden während der Reaktion gegebenenfalls unter Zuhilfenahme eines Lösungsmittels zudosiert.

Die Art der Aufarbeitung im Anschluß an das erfindungsgemäße Verfahren zur Herstellung der Verbindungen der Formel (V) durch Sauerstoffoxidation erfolgt nach den üblichen Methoden. Sie richtet sich nach den jeweiligen Ausführungsbedingungen. Wurde in Gegenwart eines Metallkatalysators gearbeitet, so ist aus Gründen den Umweltschutzes eine weitgehende Wiedergewinnung des Metalls angezeigt. Da die Oxidation im erfindungsgemäßen Verfahren in alkalischem Medium stattfindet, liegen die Metallverbindungen, soweit sie als Oxide oder Salze eingesetzt wurden, nach der Hydrolyse des Reaktionsgemisches als Oxidhydrate vor. Die Metalle können in Form ihrer Oxidhydrate beispielsweise direkt vom Reaktionsgemisch abfiltriert werden, wobei dieses bei Bedarf genügend verdünnt wurde, um Einsatzstoffe und Produkte zu lösen. Das Filtrat kann anschließend eingedampft, der Rückstand in Wasser aufgenommen und das 5-Hydroxypyrimidin durch Ansäuern freigesetzt werden.

In einer Aufarbeitungsvariante kann das Lösungsmittel aus dem Reaktionsgemisch abgedampft, der Rückstand in Wasser aufgenommen und das unlösliche Metalloxidhydrat abfiltriert werden. Dieses Vorgehen ist möglich, da sowohl das Produkt wie auch die gebildeten Nebenprodukte in wäßrig alkalischer Lösung ausgezeichnet löslich sind. Aus dem Filtrat kann wiederum das 5-Hydroxypyrimidin durch Ansäuern freigesetzt werden.

In einer noch weiteren Aufarbeitungsvariante kann das Reaktionsgemisch direkt mit Wasser versetzt und das unlösliche Oxidhydrat abfiltriert werden. Aus dem Filtrat wird das organische Lösungsmittel beispielsweise destillativ oder extraktiv abgetrennt und aus der wäßrig alkalischen Lösung das 5-Hydroxypyrimidin durch Ansäuern freigesetzt.

Ist das bei dem erfindungsgemäßen Verfahren verwendete Lösungsmittel wasserunlöslich, so kann das Reaktionsgemisch mit Wasser versetzt werden, der organische Teil abgetrennt und gegebenenfalls recyclisiert werden. Aus der wäßrig alkalischen Lösung wird das Metalloxidhydrat anschließend durch Filtration entfernt und aus dem Filtrat durch Ansäuern das 5-Hydroxypyrimidin freigesetzt.

Das nach dem Ansäuern der wäßrig alkalischen Lösung freigesetzte 5-Hydroxypyrimidin kann nach den bekannten Methoden isoliert werden, beispielsweise durch Filtration und/oder Extraktion und anschließend gereinigt werden, beispielsweise durch Destillation und/oder Kristallisation.

Als Beispiele für die erfindungsgemäß erhältlichen Verbindungen der allgemeinen Formel (V) seien aufgeführt:

Tabelle 5

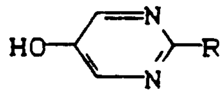

(V)

| R | R |
|---|---|
| H | $-OC_2H_5$ |
| $-CH_3$ | $-OC_3H_7$-n |
| $-C_2H_5$ | $-OC_3H_7$-i |
| $-C_3H_7$-n | $-CH_2OCH_3$ |
| $-C_3H_7$-i | $-CH_2CH_2OCH_3$ |
| H | $-OC_2H_5$ |
| $-C_4H_9$-n | $-CH_2OC_2H_5$ |
| $-C_4H_9$-i | $-CH_2CH_2OC_2H_5$ |
| $-C_4H_9$-s | $-CH_2SO_2CH_3$ |
| $-C_4H_9$-t | $-CH_2CH_2SO_2CH_3$ |
| $-C_5H_{11}$-n | $-CH_2CH_2SO_2C_2H_5$ |

Tabelle 5 - Fortsetzung

| R | R |
|---|---|
| $-C_5H_{11}$-t | $-N(CH_3)_2$ |
| $-OCH_3$ | $-N(C_2H_5)_2$ |
| 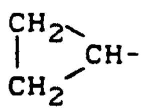 | 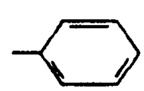 |
| 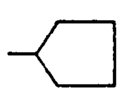 | 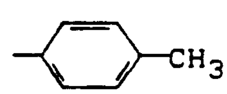 |
| 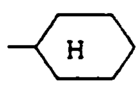 | |

Diese Verbindungen können beispielsweise in der Verfahrensstufe (d) eingesetzt werden.

In der Verfahrensstufe (d) werden die Verbindungen der allgemeinen Formel (I) aus den Verbindungen der allgemeinen Formeln (VI) und (V) erhalten.

Verwendet man in der Verfahrensstufe (d) beispielsweise O-Ethyl-O-isopropyl-thionophosphorsäurediesterchlorid und 5-Hydroxy-2-phenyl-pyrimidin als Ausgangsstoffe, so kann die entsprechende Reaktion durch das folgende Formelschema skizziert werden:

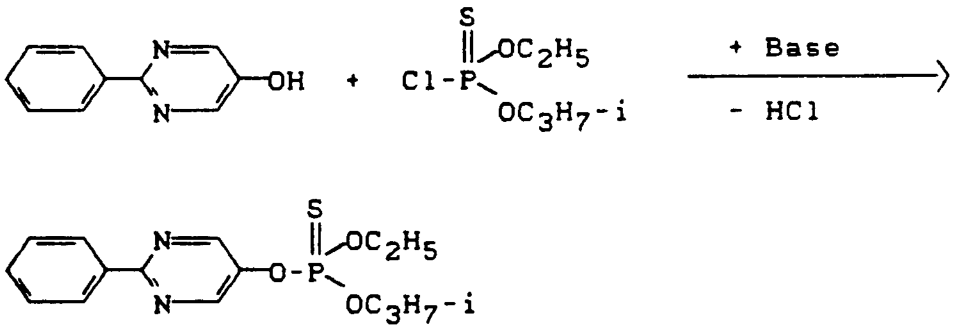


Die in der Verfahrensstufe (d) einzusetzenden Ausgangsstoffe der allgemeinen Formel (VI) sind bekannt und nach literaturbekannten Verfahren und Methoden technisch gut herstellbar. Als Beispiele dafür seien im einzelnen genannt:
O,O-Dimethyl-, O,O-Diethyl-, O,O-Di-n-propyl-, O,O-Di-iso-propyl-, O,O-Di-n-butyl-, O,O-Di-iso-butyl-, O,O-Di-sec-butyl-, O-Methyl-O-ethyl-, O-Methyl-O-n-propyl-, O-Methyl-O-iso-propyl-, O-Methyl-O-n-butyl-, O-Methyl-O-iso-butyl-, O-Methyl-O-sec-butyl-, O-Ethyl-O-n-propyl-, O-Ethyl-O-iso-propyl-, O-Ethyl-O-n-butyl-, O-Ethyl-O-sec-butyl-, O-Ethyl-O-iso-butyl-, O-n-Propyl-O-butyl- bzw. O-iso-Propyl-O-butylphosphorsäurediesterchlorid und die entsprechenden Thionoanalogen, ferner O,S-Dimethyl-, O,S-Diethyl-, O,S-Di-n-propyl-, O,S-Di-iso-propyl-, O,S-Di-n-butyl-, O,S-Di-iso-butyl-, O-Ethyl-S-n-propyl-, O-Ethyl-S-iso-propyl-, O-Ethyl-S-n-butyl-, O-Ethyl-S-sec-butyl-, O-n-Propyl-S-ethyl-, O-n-Propyl-S-iso-propyl-, O-n-Butyl-S-n-propyl- und O-sec-Butyl-S-ethylthiol-phosphorsäurediesterchlorid und die entsprechenden Thioanalogen, ferner O-Methyl-, O-Ethyl-, O-n-Propyl-, O-iso-Propyl-, O-n-Butyl-, O-iso-Butyl- bzw. O-sec-Butyl-methan- bzw. -ethan-, n-propan-, -n-butan-, -iso-butan-, -sec-butan- bzw. -phenyl-phosphonsäureesterchlorid und die entsprechenden Thionoanalogen, und O-Methyl-N-methyl-, O-Methyl-N-ethyl-, O-Methyl-N-n-propyl-, O-Methyl-N-iso-propyl-, O-Ethyl-N-methyl-, O-Ethyl-N-ethyl-, O-Ethyl-N-n-propyl-, O-Ethyl-N-iso-propyl-, O-n-Propyl-N-methyl-, O-n-Propyl-N-ethyl-, O-n-Propyl-N-n-propyl-, O-n-Propyl-N-iso-propyl-, O-iso-Propyl-N-methyl-, O-iso-Propyl-N-ethyl-, O-iso-Propyl-N-n-propyl-, O-iso-Propyl-N-iso-propyl-, O-n-Butyl-N-methyl-, O-n-Butyl-N-ethyl-, O-n-Butyl-N-n-propyl-, O-n-Butyl-N-iso-propyl-, O-iso-Butyl-N-methyl-, O-iso-Butyl-N-ethyl-, O-iso-Butyl-N-n-propyl-, O-iso-Butyl-N-iso-propyl-, O-sec-Butyl-N-methyl-, O-sec-Butyl-N-ethyl-, O-sec-Butyl-N-n-propyl-, und O-sec-Butyl-N-iso-propyl-phosphorsäuremonoesteramidchlorid und die entsprechenden Thionoanalogen.

Die Verfahrensstufe (d) zur Herstellung der Verbindungen der allgemeinen Formel (I) wird bevorzugt unter Mitverwendung geeigneter Lösungs- und Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzol, Toluol, Xylol, Benzin, Methylenchlorid, Chloroform, Tetrachlorkohlenstoff, Chlorbenzol oder Ether, wie Diethyl- und Dibutylether, Dioxan, ferner Ketone, beispielsweise Aceton, Methylethyl-, Methylisopropyl- und Methylisobutylketon, außerdem Nitrile, wie Aceto- und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, Kalium-tert-butylat, ferner aliphatische, aromatische oder heterocyclische Amine, beispielsweise Triethylamin, Trimethylamin, Dimethylanilin, Dimethylbenzylamin und Pyridin.

Die Reaktionstemperatur kann innerhalb eines größeren Bereiches variiert werden. Im allgemeinen arbeitet man zwischen 0° C und 100° C, vorzugsweise bei 20° C bis 60° C.

Die Umsetzung läßt man im allgemeinen bei Normaldruck ablaufen.

Zur Durchführung der Verfahrensvariante (d) setzt man die Ausgangsmaterialien meist in äquivalentem Verhältnis ein. Ein Überschuß der einen oder anderen Komponente bringt keine wesentlichen Vorteile. Die Reaktionspartner werden meist in einem der oben angeführten Lösungsmittel in Gegenwart eines Säurebindemittels vereinigt und bei erhöhter Temperatur zur Vervollständigung der Reaktion eine oder mehrere Stunden gerührt. Danach versetzt man die Mischung mit einem organischen Lösungsmittel, z.B. Toluol, und arbeitet die organische Phase in üblicher Weise durch Waschen, Trocknen und Abdestillieren des Lösungsmittels auf.

Die Verbindungen der allgemeinen Formel (I) fallen meist in Form von Ölen an, die sich häufig nicht unzersetzt destillieren lassen, jedoch durch sogenanntes "Andestillieren", d.h. durch längeres Erhitzen unter vermindertem Druck auf mäßig erhöhte Temperatur von den letzten flüchtigen Anteilen befreit und auf diese Weise gereinigt werden. Zu ihrer Charakterisierung dient der Brechungsindex.

Wie bereits mehrfach erwähnt, zeichnen sich die erfindungsgemäß erhältlichen Verbindungen der allgemeinen Formel (I) durch eine hervorragende insektizide, akarizide und nematizide Wirkung aus. Sie wirken gegen Pflanzen-, Hygiene- und Vorratsschädlinge und auf dem veterinärmedizinischen Sektor. Sie besitzen bei geringer Phytotoxizität sowohl eine gute Wirkung gegen saugende als auch fressende Insekten und Milben.

Aus diesem Grund können die erfindungsgemäß erhältlichen Verbindungen der allgemeinen Formel (I) mit Erfolg im Pflanzenschutz sowie auf dem Hygiene-, Vorratsschutz- und Veterinärsektor als Schädlingsbekämpfungsmittel eingesetzt werden.

Viele der erfindungsgemäß erhältlichen Verbindungen und ihre Verwendung sind bekannt und werden beschrieben z.B. in DE-OS 26 43 262, US-PS 4 127 652, EP-A 0 009 566, US-PS 4 325 948, US-PS 4 444 764 und US-PS 4 429 125.

Wie bereits oben dargelegt, ist es nach dem erfindungsgemäßen Verfahren gemäß den Verfahrensstufen (a) bis (d) möglich, die wertvollen Verbindungen der allgemeinen Formel (I) in glatten Reaktionen auf einfache Weise herzustellen, wobei hervorragende Gesamtausbeuten erhalten werden. Das erfindungsgemäße Verfahren (a) bis (d) eröffnet in überraschender Weise durch die spezielle Kombination der Verfahrensschritte und durch den teilweisen Einsatz hierbei entstehender neuer Verbindungen einen Weg, welcher eine bisher nicht erreichbare kostengünstige Herstellung der Verbindungen der allgemeinen Formel (I) erlaubt.

Da die einzelnen Zwischenprodukte stabil sind und vor allem im Falle ihrer Isolierung über längere Zeit bevorratet werden können, erlaubt das erfindungsgemäße Verfahren darüber hinaus eine außerordentliche Flexibilität in der Produktion, so daß bei rasch einsetzendem Bedarf der Endprodukte eine bedarfsgerechte Fertigung möglich ist, was insbesondere durch die klimatisch bedingten starken saisonalen Schwankungen auf dem Pflanzenschutzgebiet von sehr großer Bedeutung sein kann.

Im folgenden sollen die erfindungsgemäßen Verfahren (bzw. Verfahrensstufen) und Verbindungen durch die nachstehenden Herstellungsbeispiele erläutert werden:

Alle Prozentangaben beziehen sich auf Gewichtsprozente, wenn nicht anderes angegeben wird.

A) Verfahren zur Herstellung der Verbindungen der allgemeinen Formel (IV)

Verfahrensvariante a1):

Beispiel A 1:

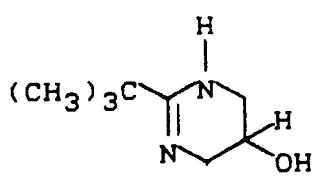

x HCl

Zu einer Lösung von 9 g (0,1 Mol) 1,3-Diamino-propanol-2 in 30 ml Ethanol gibt man portionsweise 13,6 g (0,1 Mol) Pivalamidin-Hydrochlorid. Das Gemisch wird 90 Minuten unter Rückfluß gekocht, auf Raumtemperatur abgekühlt und mit 70 ml Diethylether versetzt. Man saugt das ausgefallene Produkt ab.

Man erhält so 19 g (99 % der Theorie) 2-tert-Butyl-5-hydroxy-3,4,5,6-tetrahydropyrimidin-Hydrochlorid in Form farbloser, stark hygroskopischer Kristalle.

Beispiel A2:

Zu einer Lösung von 13,5 g (0,15 Mol) 1,3-Diamino-propanol-2 in 30 ml Ethanol gibt man portionsweise 22,8 g (0,15 Mol) Pivalyliminoethylether-Hydrochlorid und kocht die Mischung 2 Stunden unter Rückfluß. Analog zu der unter Beispiel 1 beschriebenen Aufarbeitung erhält man 28,5 g (99 % der Theorie) 2-tert-Butyl-5-hydroxy-3,4,5,6-tetrahydropyrimidin-Hydrochlorid.

Analog den Beispielen A1 und A2 unter Berücksichtigung der Angaben in der Beschreibung zu der erfindungsgemäßen Verfahrensvariante (a1) werden die nachfolgend aufgeführten Verbindungen der Formel (IV) erhalten:

Tabelle 6:

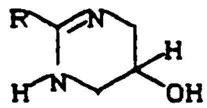 x HCl (IV)

| Bsp.-Nr. | R |
|---|---|
| A 3 | $-C_3H_7-n$ |
| A 4 | H |
| A 5 | $-CH_3$ |
| A 6 | $-N(CH_3)_2$ |
| A 7 | $-C_2H_5$ |

| Bsp.-Nr. | R | Fp[°C] |
|---|---|---|
| A 8 | 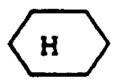 | |
| A 9 | 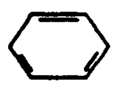 | |

Verfahrensvariante a2):

Beispiel A10

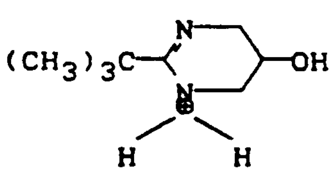 $(CH_3)_3C-CO_2^{\ominus}$

306 g (3 Mol) Pivalinsäure und 135 g (1,5 Mol) 1,3-Diamino-propanol-2 werden in 3 l Xylolgemisch 22 Stunden am Wasserabscheider gekocht. Danach sind ca. 55 ml Wasser abgeschieden. Man läßt abkühlen, saugt ab und trocknet.

Man erhält 325 g (84 % der Theorie) weiße Kristalle vom Schmelzpunkt 154° C.

Analog Beispiel A10 werden die folgenden neuen Verbindungen der Formel IV erhalten:

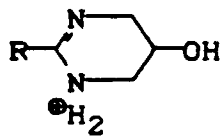 $Z^{\ominus}$

| Beispiel | R | Z | Ausbeute % der Theorie | phys. Daten |
|---|---|---|---|---|
| A11 | $(C_2H_5)_2CH-$ | $(C_2H_5)_2CHCO_2^{\ominus}$ | 77,4 | gelbes Öl |
| A12 | 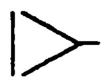 | 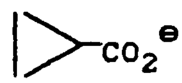 | 60,3 | rotes Öl |
| A13 | Benzyl | Benzyl-$CO_2^{\ominus}$ | 91,2 | Fp. 152° C |
| A14 | $CH_3OCH_2-$ | $CH_3OCH_2CO_2^{\ominus}$ | 78,4 | Fp. 92° C |
| A15 | $CH_3-$ | $CH_3CO_2^{\ominus}$ | 83,3 | gelbe hydros-kop. Kristalle |

Verfahrensvariante a2):

Beispiel A16

441 (3 Mol) Di-Pivalinsäuresalz des 1,3-Diamino-propanol-2

$[(CH_3)_3C-CO_2^{\ominus}]_2$ 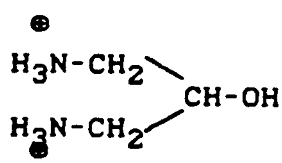 Fp. 158° C

werden in 3 l Xylolgemisch 22 Stunden am Wasserabscheider gekocht. Die Aufarbeitung erfolgt wie in Beispiel A10.

B) Verfahren zur Herstellung der Verbindungen der allgemeinen Formel (IVa)

Beispiel B1

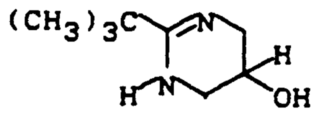

57,7 g (0,3 Mol) 2-tert-Butyl-5-hydroxy-3,4,5,6-tetrahydropyrimidin-Hydrochlorid werden in 100 ml Wasser gelöst und mit 20 ml 45-prozentiger Natronlauge versetzt. Das ausgefallene Produkt wird abgesaugt.
Man erhält so 32,7 g (70 % der Theorie) 2-tert-Butyl-5-hydroxy-3,4,5,6-tetrahydropyrimidin in Form farbloser Kristalle mit dem Schmelzpunkt 210° C.

Analog Beispiel B1 und unter Berücksichtigung der Angaben in der Beschreibung zu der erfindungsgemäßen Verfahrensstufe (b) werden die nachfolgend aufgeführten Verbindungen der Formel (IVa) erhalten:

**Tabelle 7:**

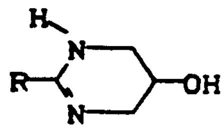

(IVa)

**Tabelle 7**

| Beispiel-Nr. | R |
|---|---|
| B2 | $-C_3H_7-n$ |
| B3 | H |
| B4 | $-CH_3$ |
| B5 | $-N(CH_3)_2$ |
| B6 | $-C_2H_5$ |
| B7 | 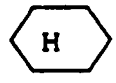 |
| B8 | 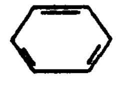 |

Beispiel B9

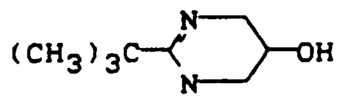

Man löst 12,9 g (0,05 Mol) Pivalinsäuresalz des 5-Hydroxy-2-tert.-butyl-tetrahydropyrimidins in 60 ml Wasser und gibt einen stark basischen Ionenaustauscher (Lewatit MP 500; Lewatit = $W_z$ der BAYER AG, Leverkusen, Bundesrepublik Deutschland) hinzu. Man rührt ca. 5 Minuten, saugt ab und engt die wäßrige Mutterlauge im Vakuum (Dampfstrahler) bei 30° C ein. Es bleiben 7,4 g 5-Hydroxy-2-tert.-butyl-tetrahydropyrimidin (freie Base) in Form von weißen Kristallen zurück. Dies entspricht einer Ausbeute von 94,9 % der Theorie.

C) Verfahren zur Herstellung der Verbindungen der allgemeinen Formel (V) [Verfahrensstufe (c)]

Beispiel C1:

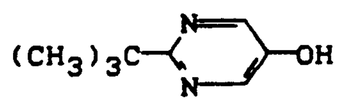

Zu 15,8 g 1,4,5,6-Tetrahydro-2-tert-butyl-5-hydroxypyrimidin in 80 ml Wasser werden portionsweise unter Kühlung innerhalb von ca. 20 Minuten 10,4 g (0,066 Mol) Kaliumpermanganat so zugetropft, daß die Reaktionstemperatur nicht über 40° C steigt. Nach der Zugabe wird eine Stunde im Wasserbad, anschließend 10 Minuten bei 90° C gerührt und dann warm abgesaugt. Das Filtrat wird bei Raumtemperatur mit konzentrierter Salzsäure auf pH 4 - 5 eingestellt, ca. eine Stunde im Eisbad stehengelassen, der kristalline Niederschlag von 5-Hydroxy-2-tert-butyl-pyrimidin abgesaugt und an der Luft getrocknet (Fp: 132° C).

Analog dem Beispiel C1 und unter Berücksichtigung der Angaben in der Beschreibung zu der erfindungsgemäßen Verfahrensstufe (c) werden die nachfolgend aufgeführten Verbindungen der Formel (V) erhalten:

**Tabelle 8:**

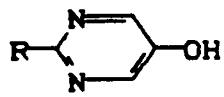 (V)

| Beispiel-Nr. | R | Fp[°C] |
|---|---|---|
| C2 | $-C_3H_7$-n | 117 |
| C3 | H | 216 |
| C4 | $-CH_3$ | 173 |
| C5 | $-N(CH_3)_2$ | 164 |
| C6 | $-C_2H_5$ | 149 |
| C7 | 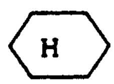 | 165 |
| C8 | 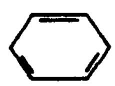 | 145 |

Beispiel C9

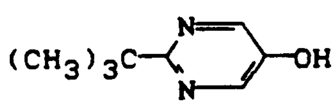

Ein Gemisch aus 5,0 g (0,032 Mol) 3,4,5,6-Tetrahydro-2-tert.-butyl-5-hydroxypyrimidin (99 %), 11,8 g (0,10 Mol) Kalium-tert.-butanolat (ca. 95 %) und 25 g t-Butanol wird bei 60° C in einer Sauerstoffatmosphäre unter Normaldruck kräftig gerührt. Der Sauerstoffverbrauch wird über eine Gasbürette gemessen. Nach 30 Minuten kommt die Sauerstoffaufnahme nach einer Gesamtaufnahme von 0,038 Mol Sauerstoff zum Stillstand.

Zur Aufarbeitung wird das Reaktionsgemisch mit 5 ml Wasser versetzt, das t-Butanol unter vermindertem Druck abdestilliert, der Rückstand in 50 ml Wasser aufgenommen und mit halbkonzentrierter Salzsäure bis pH = 4,5 angesäuert. Anschließend wird 3 x mit Diethylether extrahiert, die organische Phase über Natriumsulfat getrocknet und das Lösungsmittel abgedampft. Es bleiben 1,98 g kristalliner Rückstand mit einem Gehalt von 88,2 % 2-tert.-Butyl-5-hydroxypyrimidin, entsprechend einer Ausbeute von 35,9 %.

Beispiel C10

Durchführung wie Beispiel C9 mit dem Unterschied, daß 0,10 g Kupfer(II)oxid zugesetzt werden. Nach 20 minütiger Reaktionszeit werden 0,035 Mol Sauerstoff aufgenommen.

Zur Isolierung des Produkts wird das Reaktionsgemisch mit 5 ml Wasser hydrolysiert, das Lösungsmittel unter vermindertem Druck abdestilliert und der Rückstand in 50 ml Wasser aufgenommen. Nach einer

Filtration zur Abtrennung des Kupferoxidhydrats wird die wäßrige Lösung mit halbkonzentrierter Salzsäure bis pH = 4,5 angesäuert, mit Diethylether extrahiert, die Etherphase über Natriumsulfat getrocknet und eingedampft. Als Rückstand bleiben 3,71 g kristallines Produkt mit einem Gehalt von 89,4 % 2-tert.-Butyl-5-hydroxypyrimidin, entsprechend einer Ausbeute von 68,1 %.

Beispiel C11

Durchführung wie in Beispiel C9 mit dem Unterschied, daß 0,10 g CuO und zusätzlich 0,10 g $MnO_2$ als Katalysator zugesetzt werden. Aufarbeitung wie in Beispiel C10.

Rohprodukt 4,0 g mit einem Gehalt von 91,3 % 2-tert.-Butyl-5-hydroxypyrimidin, entsprechend einer Ausbeute von 75,0 %.

Beispiel C12

Durchführung wie Beispiel C9 mit dem Unterschied, daß 0,10 g Kobalt(II)chlorid zugesetzt werden. Aufarbeitung wie in Beispiel C10. Ausbeute 49,7 %.

Beispiel C13

Ein Gemisch aus 6,50 g (0,025 Mol) des Salzes aus 3,4,5,6-Tetrahydro-2-tert.-butyl-5-hydroxypyrimidin und Pivalinsäure, 14,5 g (0,123 Mol) Kalium-tert.-butanolat (0,95 %) und 40 g t-Butanol und 0.08 g Kupfer-(II)-oxid wird bei 60° C in einer Sauerstoffatmosphäre unter Normaldruck kräftig gerührt. Nach 30 minütiger Reaktionszeit ist die Sauerstoffaufnahme bei einer Gesamtaufnahme von 0,31 Mol beendet.

Aufarbeitung wie in Beispiel C10.

Rohprodukt 5,70 g mit einem Gehalt von 45,5 % 2-tert.-Butyl-5-hydroxypyrimidin, entsprechend einer Ausbeute von 67,7 %.

Beispiel C14

Eine Mischung aus 5,0 g (0,032 Mol) 3,4,5,6-Tetrahydro-2-tert.-butyl-5-hydroxypyrimidin (99 %ig), 4,0 g Natriumhydroxid (0,10 Mol) 25 g Dimethylsulfoxid und 0,10 g Kupfer(II)-oxid wird bei 60° C in einer Sauerstoffatmosphäre bei Normaldruck kräftig gerührt. Nach 60 Minuten ist die Sauerstoffaufnahme bei einer Gesamtaufnahme von 0,039 Mol vollständig.

Zur Produktisolierung wird das Dimethylsulfoxid unter Wärmezufuhr im Vakuum möglichst vollständig abgedampft, der feste Rückstand anschließend in 50 ml Wasser aufgenommen und die wäßrig alkalische Lösung zur Abtrennung des Kupferoxidhydrats filtriert. Das Filtrat wird bis pH = 4,5 angesäuert, mit Diethylether extrahiert, die Etherphase über Natriumsulfat getrocknet und eingedampft. Als Rückstand bleiben 4,15 g kristallines Produkt mit einem Gehalt von 88,1 % 2-tert.-Butyl-5-hydroxypyrimidin, entsprechend einer Ausbeute von 75,1 %.

Beispiel C15

Eine Mischung aus 5,0 g (0,032 Mol) 3,4,5,6-Tetrahydro-2-tert.-butyl-5-hydroxypyrimidin (99 %), 6,6 g (0,10 Mol) Kaliumhydroxid (ca. 85 %), 40 g $\gamma$-Picolin, 0,1 g Kupferoxid und 0,1 g Mangandioxid wird in einer Sauerstoffatmosphäre bei 90° C unter Normaldruck kräftig gerührt. Während 4 Stunden werden 0,038 Mol Sauerstoff aufgenommen.

Aufarbeitung wie in Beispiel C10.

Rohprodukt 3,05 g mit einem Gehalt von 68,3 % 2-tert.-Butyl-5-hydroxypyrimidin. Ausbeute 42,8 %.

Beispiel C16

Eine Mischung aus 5,0 g (0,032 Mol) 3,4,5,6-Tetrahydro-2-tert.-butyl-5-hydroxypyrimidin (99 %), 11,8 g (0,10 Mol) Kalium-tert.-butanolat (95 %), 40 g Dimethylformamid und 0,10 g Kupfer(II)oxid wird in einer Sauerstoffatmosphäre bei 60° C gerührt. Nach 15 Minuten ist die Sauerstoffaufnahme bei einer Gesamtaufnahme von 0,036 Mol beendet.

Aufarbeitung wie in Beispiel C10.

Ausbeute 55,9 %

D) Verfahren zur Herstellung der Verbindungen der allgemeinen Formel (I) [Verfahrensvariante (d)]

Beispiel D1

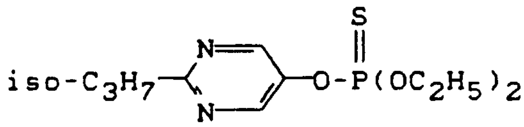

Ein Gemisch aus 300 ml Acetonitril, 13,8 g (0,1 Mol) 2-iso-Propyl-5-hydroxy-pyrimidin, 20,7 g (0,15 Mol) Kaliumcarbonat und 18,8 g (0,1 Mol) O,O-Diethyl-thionophosphorsäurediesterchlorid wird 2 Stunden bei 45 ° C gerührt. Dann gießt man das Reaktionsgemisch in 400 ml Toluol und wäscht es zweimal mit je 300 ml Wasser. Die Toluollösung wird über Natriumsulfat getrocknet und im Vakuum eingedampft. Den Rückstand destilliert man im Hochvakuum an.

Man erhält so 17,4 g (62 % der Theorie) O,O-Diethyl-O-[2-iso-propyl-pyrimidin-5-yl]-thionophosphorsäureester in Form eines braunen Öles mit dem Brechungsindex $n_D^{21}$ = 1,4970.

In analoger Weise können die folgenden Verbindungen der Formel (I)

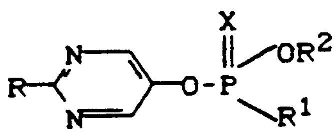

(I)

hergestellt werden:

**Tabelle 9:**

| Bsp. Nr. | $R^2$ | $R^1$ | R | X | Ausbeute (% d.Th.) | Brechungsindex |
|---|---|---|---|---|---|---|
| D2 | $C_3H_7$-i | $CH_3$ | $C_3H_7$-i | S | 74 | $n_D^{21}$: 1,5102 |
| D3 | $CH_3$ | $OCH_3$ | $C_3H_7$-i | S | 66 | $n_D^{24}$: 1,5080 |
| D4 | $C_2H_5$ | $SC_3H_7$-n | $C_3H_7$-i | S | 69 | $n_D^{26}$: 1,5284 |
| D5 | $C_2H_5$ | 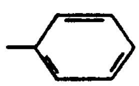 | $C_3H_7$-i | S | 74 | $n_D^{26}$: 1,5570 |
| D6 | $C_2H_5$ | $OC_2H_5$ | $C_3H_7$-i | O | 82 | $n_D^{32}$: 1,4630 |
| D7 | $C_2H_5$ | NH-$C_3H_7$-i | $C_3H_7$-i | S | 57 | $n_D^{32}$: 1,5057 |
| D8 | $C_3H_7$-n | $OC_2H_5$ | $C_3H_7$-i | S | 73 | $n_D^{32}$: 1,4929 |
| D9 | $C_2H_5$ | $OC_2H_5$ | $CH_3$ | S | 92 | $n_D^{32}$: 1,4992 |
| D10 | $C_2H_5$ | $C_2H_5$ | $CH_3$ | S | 80 | $n_D^{32}$: 1,5169 |

Tabelle 9 - Fortsetzung

| Bsp. Nr. | $R^2$ | $R^1$ | R | X | Ausbeute (% d.Th.) | Brechungsindex |
|---|---|---|---|---|---|---|
| D11 | $C_2H_5$ | $OC_2H_5$ | 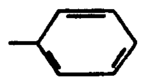 | S | 80 | $n_D^{32}$:1,5643 |
| D12 | $C_2H_5$ | $C_2H_5$ | 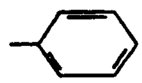 | S | 80 | $n_D^{32}$:1,5827 |
| D13 | $C_2H_5$ | $OC_2H_5$ | H | S | 72 | $n_D^{32}$:1,5028 |
| D14 | $C_2H_5$ | $OC_2H_5$ | $-C_2H_5$ | S | 84 | $n_D^{20}$:1,5014 |
| D15 | $C_2H_5$ | $OC_2H_5$ | $-C_3H_7$-n | S | 60 | $n_D^{26}$:1,4833 |
| D16 | $C_2H_5$ | $OC_2H_5$ | $-C_4H_9$-n | S | 94 | $n_D^{21}$:1,4958 |
| D17 | $C_2H_5$ | $OC_2H_5$ | $-C_4H_9$-t | S | 61 | $n_D^{26}$:1,4902 |
| D18 | $C_2H_5$ | $OC_2H_5$ | 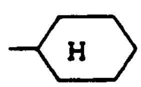 | S | 66 | $n_D^{23}$:1,5158 |
| D19 | $C_2H_5$ | $-NHOC_3H_7$-i | 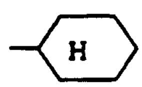 | S | 51 | $n_D^{23}$:1,5246 |
| D20 | $CH_3$ | $-OCH_3$ | 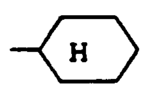 | S | 64 | $n_D^{23}$:1,5287 |

Tabelle 9 -Fortsetzung

| Bsp. Nr. | $R^2$ | $R^1$ | R | X | Ausbeute (% d.Th.) | physikal. Daten [Brechungsindex/ Schmelzpunkt °C] |
|---|---|---|---|---|---|---|
| D21 | $C_2H_5$ | $OC_2H_5$ | 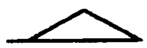 | S | 78 | $n_D^{24}$ :1,5142 |
| D22 | $C_2H_5$ | $NHC_3H_7$-i | 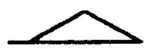 | S | 62 | 49 |
| D23 | $CH_3$ | $OCH_3$ | 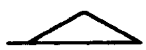 | S | 43 | $n_D^{24}$ :1,5390 |
| D24 | $C_3H_7$-n | $OC_2H_5$ | 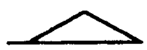 | S | 71 | $n_D^{25}$ :1,5128 |
| D25 | $C_2H_5$ | $NHC_2H_5$ | 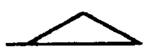 | S | 74 | $n_D^{26}$ :1,5310 |
| D26 | $C_2H_5$ | $OC_2H_5$ | 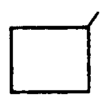 | S | | |
| D27 | $C_2H_5$ | $OC_2H_5$ | 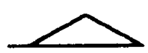 | S | | |
| D28 | $C_2H_5$ | $OC_2H_5$ | 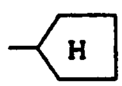 | S | 80 | $n_D^{23}$ :1,5164 |
| D29 | $C_2H_5$ | $OC_3H_7$-n | 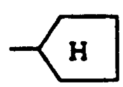 | | | |

Tabelle 9 -Fortsetzung

| Bsp. Nr. | $R^2$ | $R^1$ | R | X | Ausbeute (% d.Th.) | physikal. Daten [Brechungsindex/ Schmelzpunkt °C] |
|---|---|---|---|---|---|---|
| D30 | $C_2H_5$ | $CH_3$ | 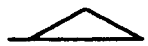 | S | 72 | $n_D^{25}$:1,5428 |
| D31 | $C_2H_5$ | $OC_2H_5$ | 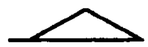 | O | | |
| D32 | $C_2H_5$ | $NHC_3H_7$-i | 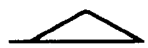 | O | | |
| D33 | $C_2H_5$ | 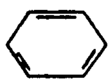 | 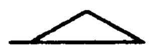 | S | 74 | $n_D^{25}$:1,5815 |
| D34 | $C_2H_5$ | $SC_3H_7$-n | 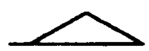 | S | | |
| D35 | $C_2H_5$ | 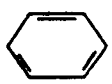 | H | S | | |
| D36 | $C_2H_5$ | $NHC_2H_5$ | H | S | 66 | $n_D^{23}$:1,5329 |
| D37 | $C_2H_5$ | $SC_3H_7$ | 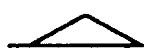 | O | | |
| D38 | $C_2H_5$ | $C_2H_5$ | 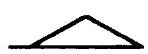 | S | | |

<u>Tabelle 9</u> -Fortsetzung

| Bsp. Nr. | $R^2$ | $R^1$ | R | X | Ausbeute (% d.Th.) | physikal. Daten [Brechungsindex/ Schmelzpunkt °C] |
|---|---|---|---|---|---|---|
| D39 | $CH_3$ | $C_2H_5$ | 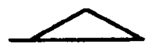 | S | | |
| D40 | $C_3H_7$-i | $CH_3$ | 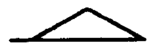 | S | 67 | $n_D^{26}$ :1,5233 |
| D41 | $CH_3$ | $NHC_3H_7$-i | 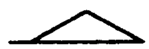 | S | | |
| D42 | $CH_3$ | $NHCH_3$ | 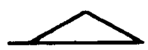 | S | 66 | $n_D^{26}$ :1,5460 |
| D43 | $C_2H_5$ | $NHCH_3$ | 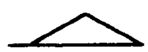 | S | | |
| D44 | $CH_3$ | $NHC_2H_5$ | 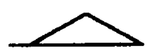 | S | | |
| D45 | $C_2H_5$ | NH-$C_3H_7$-i | 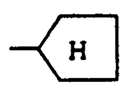 | S | 55 | $n_D^{23}$ :1,5247 |
| D46 | $C_2H_5$ | $OC_2H_5$ | 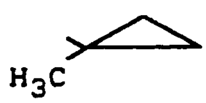 | S | | |
| D47 | $C_2H_5$ | $OC_3H_7$-i | -$C_3H_7$-i | S | 92 | $n_D^{23}$ :1,4910 |

Tabelle 9 -Fortsetzung

| Bsp. Nr. | $R^2$ | $R^1$ | R | X | Ausbeute (% d.Th.) | physikal. Daten [Brechungsindex/ Schmelzpunkt °C] |
|---|---|---|---|---|---|---|
| D48 | $C_3H_7$-i | $C_3H_7$-i | -$OC_3H_7$-i | S | | $n_D^{20}$ :1,4869 |
| D49 | $C_4H_9$-t | $C_2H_5$ | -$OC_3H_7$-i | S | | $n_D^{20}$ :1,4917 |
| D50 | $C_3H_7$-i | $C_2H_5$ | -$OC_4H_9$-s | S | | $n_D^{20}$ :1,4960 |
| D51 | $C_4H_9$-t | $C_2H_5$ | -$OC_4H_9$-s | S | | $n_D^{22}$ :1,4935 |
| D52 | $C_4H_9$-t | $C_3H_7$-i | -$OC_3H_7$-i | S | | $n_D^{22}$ :1,4857 |
| D53 | 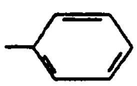 | $C_2H_5$ | -$OC_3H_7$-i | S | | $n_D^{22}$ :1,5516 |
| D54 | $C_4H_9$-t | $C_2H_5$ | -$NHC_2H_5$ | S | | $n_D^{21}$ :1,5100 |
| D55 | 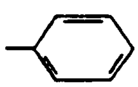 | $C_2H_5$ | -$OC_4H_9$-s | S | | |
| D56 | 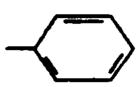 | $C_3H_7$-i | -$OC_3H_7$-i | S | | |
| D57 | $C_3H_7$-i | $C_3H_7$-n | -$OC_3H_7$-n | S | | $n_D^{23}$ :1,4915 |

## Patentansprüche

1. Verfahren zur Herstellung der Verbindungen der allgemeinen Formel (I)

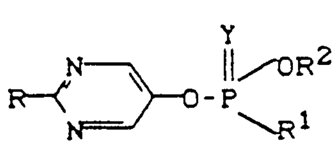

(I)

in welcher

R für Wasserstoff, für $C_1$-$C_{12}$-Alkoxy, für Mono- oder Di-$C_1$-$C_6$-Alkyl-amino, für gegebenenfalls durch $C_1$-$C_4$-Alkoxy oder $C_1$-$C_4$-Alkylsulfonyl substituiertes $C_1$-$C_{12}$-Alkyl, für gegebenenfalls durch $C_1$-$C_4$-Alkyl substituiertes $C_3$-$C_8$-Cycloalkyl und für gegebenenfalls durch $C_1$-$C_4$-Alkyl, $C_1$-$C_4$-Alkoxy oder $C_1$-$C_4$-Alkylsulfonyl substituiertes $C_6$-$C_{10}$-Aryl steht,

$R^1$ für gegebenenfalls durch $C_1$-$C_4$-Alkyl, $C_1$-$C_4$-Alkoxy, $C_1$-$C_4$-Alkylthio, Halogen, Cyano und/oder Nitro substituierte Reste aus der Reihe $C_1$-$C_6$-Alkoxy, $C_1$-$C_6$-Alkylthio, Mono- oder Di-$C_1$-$C_6$-Alkyl-amino und Phenyl oder für gegebenenfalls durch $C_1$-$C_4$-Alkoxy, $C_1$-$C_4$-Alkylthio, Halogen, Cyano und/oder Nitro substituiertes $C_1$-$C_6$-Alkyl steht,

$R^2$ für gegebenenfalls durch $C_1$-$C_4$-Alkoxy, $C_1$-$C_4$-Alkylthio, Halogen, Cyano und/oder Nitro substituiertes $C_1$-$C_6$-Alkyl steht und

Y für Sauerstoff oder Schwefel steht,

dadurch gekennzeichnet, daß man

a1) Verbindungen der allgemeinen Formel (IIa)

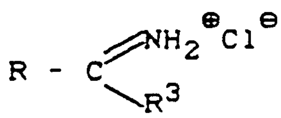

(IIa)

in welcher

R die oben angegebene Bedeutung hat,

$R^3$ für Amino oder die Gruppe $XR^4$ steht, wobei

X für Sauerstoff oder Schwefel steht und

$R^4$ für Methyl oder Ethyl steht,

mit 1,3-Diamino-2-propanol der Formel (III)

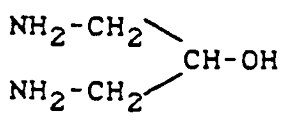

(III)

oder dessen Säureadditionssalzen,

gegebenenfalls in Gegenwart von Verdünnungsmitteln bei Temperaturen zwischen 0 °C und 120 °C umsetzt

a2) oder wenn man Carbonsäuren der Formel (IIb)

$R^5$-$CO_2H$ (IIb)

in welcher

$R^5$ die oben bei R angegebene Bedeutung, ausgenommen Wasserstoff, Alkoxy, Mono- und Dialkylamino, hat,

mit 1,3-Diamino-2-propanol der Formel (III)

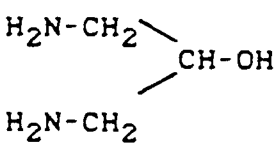

(III)

gegebenenfalls in Gegenwart von Verdünnungsmitteln bei Temperaturen zwischen 120° C und 250° C umsetzt; oder

a3) wenn man Carbonsäuresalze des 1,3-Diamino-2-propanol der Formel (IIc)

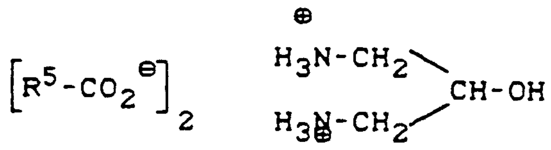

(IIc)

in welcher

$R^5$ die oben angegebene Bedeutung hat auf Temperaturen zwischen 120° C und 250° C erhitzt,

und die sich bildenden 5-Hydroxy-3,4,5,6-tetrahydropyrimidin-Salze der allgemeinen Formel (IV)

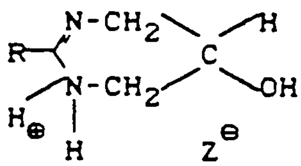

(IV)

in welcher

Z für Chlor oder $R^5$-$CO_2$ steht wobei $R^5$ die oben angegebene Bedeutung hat und

R die oben angegebene Bedeutung hat

gegebenenfalls isoliert und

b) gegebenenfalls daraus mit einer Base die 5-Hydroxy-3,4,5,6-tetrahydropyrimidine der allgemeinen Formel (IVa)

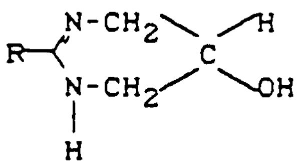

(IVa)

in welcher

R die oben angegebene Bedeutung hat,

freisetzt und anschließend

c) entweder

die 5-Hydroxy-3,4,5,6-tetrahydropyrimidin Salze der allgemeinen Formel (IV)

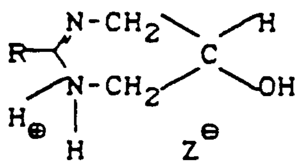

(IV)

in welcher

Z für Chlor oder $R^5$-$CO_2$ steht, wobei $R^5$ die oben angegebene Bedeutung hat und

R die oben angegebene Bedeutung hat

oder die freien 5-Hydroxy-3,4,5,6-tetrahydropyrimidine der allgemeinen Formel (IVa)

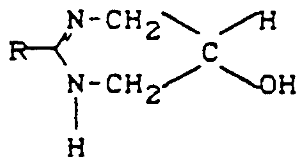

(IVa)

in welcher

R die oben angegebene Bedeutung hat,

gegebenenfalls nach ihrer Isolierung zu den Verbindungen der allgemeinen Formel (V)

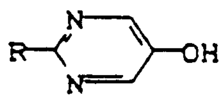

(V)

in welcher

die oben angegebene Bedeutung hat

oxidiert bzw. dehydriert und anschließend

d) die Verbindungen der Formel (V), gegebenenfalls nach ihrer Isolierung, mit Verbindungen der allgemeinen Formel (VI)

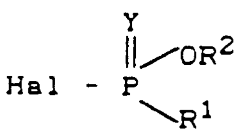

(VI)

in welcher

Hal für Halogen steht und

Y, $R^1$ und $R^2$ die oben angegebene Bedeutung haben,

gegebenenfalls in Gegenwart eines Säurebindemittels und gegebenenfalls in Gegenwart eines Lösungsmittels, umsetzt und die Verbindungen der allgemeinen Formel (I) isoliert.

**2.** Verfahren gemaß Anspruch 1, wobei in Formel (I)

R für Wasserstoff, Alkoxy mit 1 bis 6 Kohlenstoffatomen, Mono- oder Dialkylamino mit jeweils 1 bis 4 Kohlenstoffatomen im Alkylteil oder für gegebenenfalls durch Methoxy, Ethoxy, Methylsulfonyl oder Ethylsulfonyl substituiertes Alkyl mit 1 bis 6 Kohlenstoffatomen, für gegebenenfalls durch Methyl oder Ethyl substituiertes Cycloalkyl mit 3 bis 6 Kohlenstoffatomen, und für gegebenenfalls durch Methyl, Ethyl, Methoxy, Ethoxy, Methylsulfonyl oder Ethylsulfonyl substituiertes Phenyl steht.

**3.** Verfahren gemäß Anspruch 1, wobei in Formel (I)

R für Methyl, Isopropyl oder tert.-Butyl steht.

**4.** Verfahren, gemäß den Ansprüchen 1 bis 3, wobei in Formel (I)

$R^1$ Ethoxy,

$R^2$ für Ethyl oder s-Butyl und

Y für Schwefel stehen.

**5.** Verfahren zur Herstellung der 5-Hydroxy-3,4,5,6-tetrahydropyrimidin-Salze der allgemeinen Formel (IV)

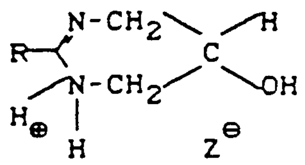
(IV)

sowie deren freien Basen (Formel IVa)
in welcher
Z und R die in Anspruch 1 angegebene Bedeutung haben,
ausgenommen die Verbindungen, in welchen R Wasserstoff, Methyl oder Chlormethyl und Z Chlor bedeutet,
dadurch gekennzeichnet, daß man
a1) Verbindungen der allgemeinen Formel (IIa)

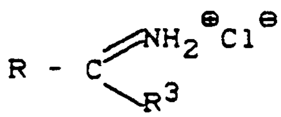
(IIa)

in welcher
R die oben angegebene Bedeutung hat,
$R^3$ für Amino oder die Gruppe $XR^4$ steht, wobei
X für Sauerstoff oder Schwefel steht und
$R^4$ für Methyl oder Ethyl steht,
mit 1,3-Diamino-2-propanol der Formel (III)

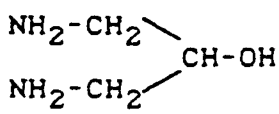
(III)

oder dessen Säureadditionssalzen,
gegebenenfalls in Gegenwart von Verdünnungsmitteln bei Temperaturen zwischen 0° C und 120° C umsetzt oder
a2) Carbonsäuren der Formel (IIb)

$R^5$-$CO_2H$ (IIb)

in welcher
$R^5$ die in Anspruch 1 angegebene Bedeutung hat,
mit 1,3-Diamino-2-propanol der Formel (III)

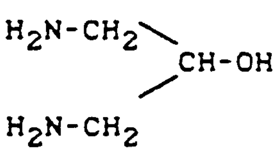
(III)

gegebenenfalls in Gegenwart von Verdünnungsmitteln bei Temperaturen zwischen 120° C und 250° C umsetzt;

oder

a3) Carbonsäuresalze des 1,3-Diamino-2-propanol der Formel (IIc)

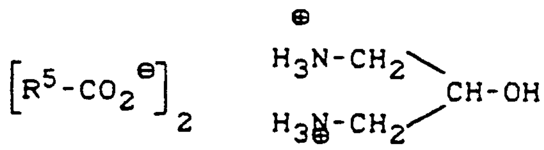 (IIc)

in welcher

$R^5$ die in Anspruch 1 angegebene Bedeutung hat, auf Temperaturen zwischen 120° C und 250° C erhitzt,

und gegebenenfalls aus den erhaltenen 5-Hydroxy-3,4,5,6-tetrahydropyrimidin-Salzen mit einer Base in üblicher weise die Basen (Formel IVa) freisetzt.

**6.** Verfahren zur Herstellung der Verbindungen der allgemeinen Formel (V)

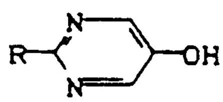 (V)

in welcher

R die in Anspruch 1 angegebene Bedeutung hat, dadurch gekennzeichnet, daß man die 5-Hydroxy-3,4,5,6-tetrahydropyrimidin-Salze der allgemeinen Formel (IV)

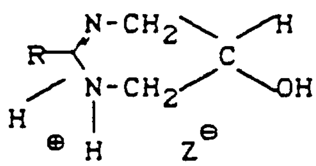 (IV)

in welcher

Z und R die in Anspruch 1 angegebene Bedeutung haben,

oder die freien 5-Hydroxy-3,4,5,6-tetrahydropyrimidine der allgemeinen Formel (IVa)

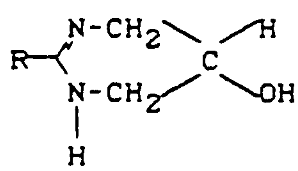 (IVa)

in welcher

R die in Anspruch 1 angegebene Bedeutung hat,

gegebenenfalls nach ihrer Isolierung, zu den Verbindungen der allgemeinen Formel (V)

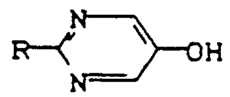

(V)

in welcher

R die in Anspruch 1 angegebene Bedeutung hat,

oxidiert bzw. dehydriert.

**7.** Verfahren gemäß Anspruch 7, dadurch gekennzeichnet, daß die Oxidation bzw. Dehydrierung in Gegenwart eines Lösungsmittels und einer Base und gegebenenfalls in Gegenwart einer Übergangsmetallverbindung bei Temperaturen zwischen 0° C und 300° C mit Sauerstoff oder Sauerstoff enthaltenden Gasen durchgeführt wird.

## Claims

**1.** Process for the preparation of the compounds of the general formula (I)

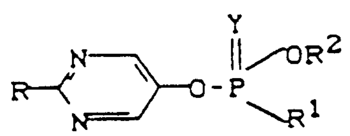

(I)

in which

R represents hydrogen, $C_1$-$C_{12}$-alkoxy, mono- or di-$C_1$-$C_6$-alkyl-amino, $C_1$-$C_{12}$-alkyl which is optionally substituted by $C_1$-$C_4$-alkoxy or $C_1$-$C_4$-alkylsulphonyl, $C_3$-$C_8$-cycloalkyl which is optionally substituted by $C_1$-$C_4$-alkyl, and $C_6$-$C_{10}$-aryl which is optionally substituted by $C_1$-$C_4$-alkyl, $C_1$-$C_4$-alkoxy or $C_1$-$C_4$-alkylsulphonyl,

$R^1$ represents radicals from the series consisting of $C_1$-$C_6$-alkoxy, $C_1$-$C_6$-alkylthio, mono- or di-$C_1$-$C_6$-alkyl-amino and phenyl, each of which is optionally substituted by $C_1$-$C_4$-alkyl, $C_1$-$C_4$-alkoxy, $C_1$-$C_4$-alkylthio, halogen, cyano and/or nitro, or $C_1$-$C_6$-alkyl which is optionally substituted by $C_1$-$C_4$-alkoxy, $C_1$-$C_4$-alkylthio, halogen, cyano and/or nitro,

$R^2$ represents $C_1$-$C_6$-alkyl which is optionally substituted by $C_1$-$C_4$-alkoxy, $C_1$-$C_4$-alkylthio, halogen, cyano and/or nitro, and

Y represents oxygen or sulphur,

characterized in that

a1) compounds of the general formula (IIa)

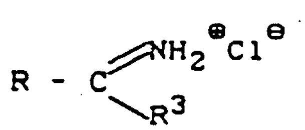

(IIa)

in which

R has the abovementioned meaning,

$R^3$ represents amino or the group $XR^4$,

where

X represents oxygen or sulphur and

$R^4$ represents methyl or ethyl,

are reacted with 1,3-diamino-2-propanol of the formula (III)

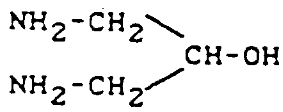 (III)

or its acid addition salts,
if appropriate in the presence of diluents at temperatures between 0 °C and 120 °C; or
a2) carboxylic acids of the formula (IIb)

$R^5$-$CO_2H$ (IIb)

in which
$R^5$ has the meaning given above for R, excluding hydrogen, alkoxy, mono- and dialkylamino,
are reacted with 1,3-diamino-2-propanol of the formula (III)

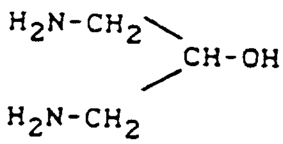 (III)

if appropriate in the presence of diluents, at temperatures between 120 °C and 250 °C; or
a3) carboxylic acid salts of 1,3-diamino-2-propanol of the formula (IIc)

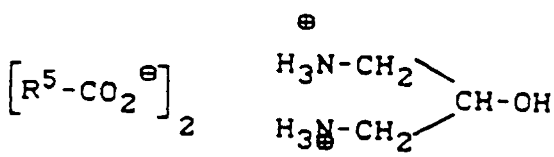 (IIc)

in which
$R^5$ has the abovementioned meaning, are heated to temperatures between 120 °C and 250 °C,
and the 5-hydroxy-3,4,5,6-tetrahydropyrimidine salts formed of the general formula (IV)

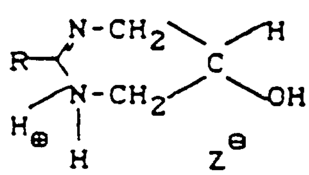 (IV)

in which
Z represents chlorine or $R^5$-$CO_2$, where $R^5$ has the abovementioned meaning, and
R has the abovementioned meaning
are optionally isolated and
b) the 5-hydroxy-3,4,5,6-tetrahydropryrimidines of the general formula (IVa)

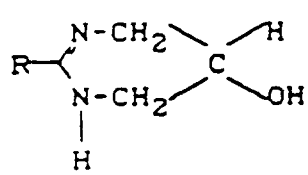 (IVa)

in which

R has the abovementioned meaning,

are optionally released therefrom using a base, and subsequently

c) either

the 5-hydroxy-3,4,5,6-tetrahydropyrimidine salts of the general formula (IV)

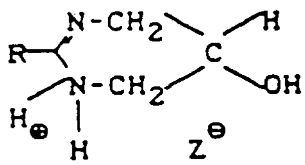

(IV)

in which

Z represents chlorine or $R^5$-$CO_2$, where $R^5$ has the abovementioned meaning, and

R has the abovementioned meaning

or the free 5-hydroxy-3,4,5,6-tetrahydropyrimidines of the general formula (IVa)

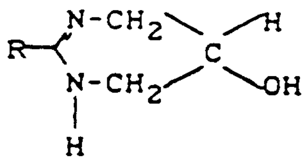

(IVa)

in which

R has the abovementioned meaning,

are oxidized or dehydrogenated, optionally after their isolation, to give the compounds of the general formula (V)

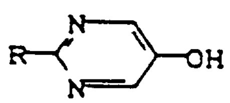

(V)

in which

R has the abovementioned meaning, and subsequently

d) the compounds of the formula (V), optionally after their isolation, are reacted with compounds of the general formula (VI)

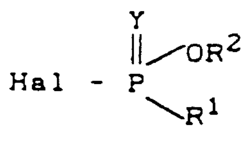

(VI)

in which

Hal represents halogen and

Y, $R^1$ and $R^2$ have the abovementioned meaning,

if appropriate in the presence of an acid-binding agent and if appropriate in the presence of a solvent, and the compounds of the general formula (I) are isolated.

**2.** Process according to Claim 1, wherein in formula (I)

R represents hydrogen, alkoxy having 1 to 6 carbon atoms, mono- or dialkylamino each having 1 to 4 carbon atoms in the alkyl moiety or for alkyl having 1 to 6 carbon atoms which is optionally substituted by methoxy, ethoxy, methylsulphonyl or ethylsulphonyl, cycloalkyl having 3 to 6 carbon atoms which is optionally substituted by methyl or ethyl, and phenyl

which is optionally substituted by methyl, ethyl, methoxy, ethoxy, methylsulphonyl or ethylsulphonyl.

3. Process according to Claim 1, wherein in formula (I)
R represents methyl, isopropyl or tert.-butyl.

4. Process, according to Claims 1 to 3, wherein in formula (I)
$R^1$ represents ethoxy,
$R^2$ represents ethyl or s-butyl and
Y represents sulphur.

5. Process for the preparation of the 5-hydroxy-3,4,5,6-tetrahydropyrimidine salts of the general formula (IV)

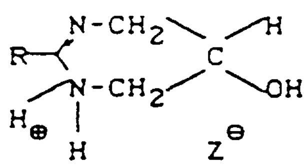

(IV)

and their free bases (formula IVa)
in which
Z and R have the meaning given in Claim 1,
excluding the compounds in which R denotes hydrogen, methyl or chloromethyl and Z denotes chlorine,
characterized in that
a1) compounds of the general formula (IIa)

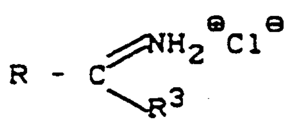

(IIa)

in which
R has the abovementioned meaning,
$R^3$ represents amino or the group $XR^4$,
where
X represents oxygen or sulphur and
$R^4$ represents methyl or ethyl,
are reacted with 1,3-diamino-2-propanol of the formula (III)

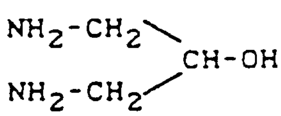

(III)

or its acid addition salts
if appropriate in the presence of diluents at temperatures between 0 °C and 120 °C, or
a2) carboxylic acids of the formula (IIb)

$R^5$-$CO_2H$ (IIb)

in which
$R^5$ has the meaning given in Claim 1,
are reacted with 1,3-diamino-2-propanol of the formula (III)

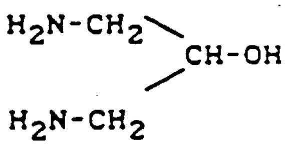

(III)

if appropriate in the presence of diluents, at temperatures between 120 ° C and 250 ° C;
or
a3) carboxylic acid salts of 1,3-diamino-2-propanol of the formula (IIc)

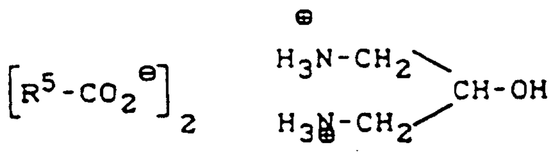

(IIc)

in which

$R^5$ has the meaning given in Claim 1, are heated to temperatures between 120 ° C and 250 ° C,

and the bases (formula IVa) are optionally released from the 5-hydroxy-3,4,5,6-tetrahydropyrimidine salts obtained using a base.

**6.** Process for the preparation of the compounds of the general formula (V)

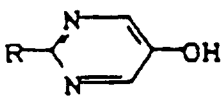

(V)

in which

R has the meaning given in Claim 1, characterized in that the 5-hydroxy-3,4,5,6-tetrahydropyrimidine salts of the general formula (IV)

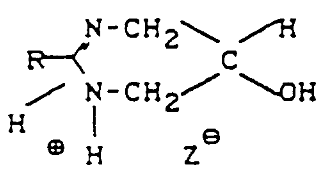

(IV)

in which

Z and R have the meaning given in Claim 1,

or the free 5-hydroxy-3,4,5,6-tetrahydropyrimidines of the general formula (IVa)

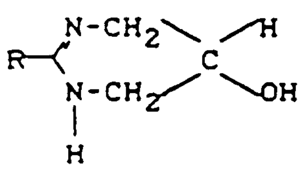

(IVa)

in which

R has the meaning given in Claim 1, are oxidized or dehydrated, if appropriate after their isolation, to give the compounds of the general formula (V)

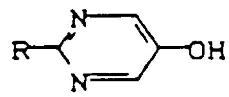

(V)

in which

R has the meaning given in Claim 1.

**7.** Process according to Claim 7, characterized in that the oxidation or dehydration is carried out in the presence of a solvent and of a base and if appropriate in the presence of a transition metal compound at temperatures between 0 °C and 300 °C using oxygen or oxygen-containing gases.

## Revendications

**1.** Procédé pour la préparation des composés répondant à la formule générale (I)

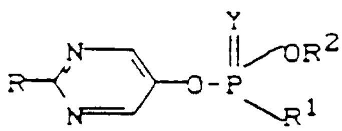

(I)

dans laquelle

R représente un atome d'hydrogène, un groupe alcoxy en $C_1$-$C_{12}$, un groupe mono- ou dialkyl-(en $C_1$-$C_6$)amino, un groupe alkyle en $C_1$-$C_{12}$ éventuellement substitué par un groupe alcoxy en $C_1$-$C_4$ ou par un groupe alkyl(en $C_1$-$C_4$)sulfonyle, un groupe cycloalkyle en $C_3$-$C_8$ éventuellement substitué par un groupe alkyle en $C_1$-$C_4$, et un groupe aryle en $C_6$-$C_{10}$ éventuellement substitué par un groupe alkyle en $C_1$-$C_4$, par un groupe alcoxy en $C_1$-$C_4$ ou par un groupe alkyl(en $C_1$-$C_4$)sulfonyle,

$R^1$ représente des radicaux de la série comprenant un groupe alcoxy en $C_1$-$C_6$, un groupe alkyl-(en $C_1$-$C_6$)thio, un groupe mono- ou dialkyl(en $C_1$-$C_6$)amino et un groupe phényle éventuellement substitué par un groupe alkyle en $C_1$-$C_4$, par un groupe alcoxy en $C_1$-$C_4$, par un groupe alkyl(en $C_1$-$C_4$)thio, par un atome d'halogène, par un groupe cyano et/ou par un groupe nitro, ou encore représente un groupe alkyle en $C_1$-$C_6$ éventuellement substitué par un groupe alcoxy en $C_1$-$C_4$, par un groupe alkyl(en $C_1$-$C_4$)thio, par un atome d'halogène, par un groupe cyano et/ou par un groupe nitro,

$R^2$ représente un groupe alkyle en $C_1$-$C_6$ éventuellement substitué par un groupe alcoxy en $C_1$-$C_4$, par un groupe alkyl(en $C_1$-$C_4$)thio, par un atome d'halogène, par un groupe cyano et/ou par un groupe nitro,

Y représente un atome d'oxygène ou un atome de soufre,

caractérisé en ce que

a1) on fait réagir des composés répondant à la formule générale (IIa)

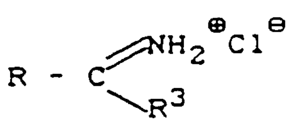

(IIa)

dans laquelle

R a la signification indiquée ci-dessus

$R^3$ représente un groupe amino ou le groupe $XR^4$ où

X représente un atome d'oxygène ou un atome de soufre et

$R^4$ représente un groupe méthyle ou un groupe éthyle,

avec le 1,3-diamino-2-propanol de formule (III)

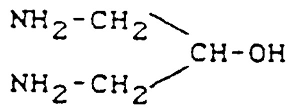

(III)

ou avec ses sels d'addition d'acides, éventuellement en présence de diluants, à des températures entre 0 °C et 120 °C

a2) ou bien lorsqu'on fait réagir des acides carboxyliques de formule (IIb)

$R^5$-$CO_2H$ (IIb)

dans laquelle

$R^5$ a la signification indiquée ci-dessus pour R, à l'exclusion d'un atome d'hydrogène, d'un groupe alcoxy, d'un groupe mono- et dialkylamino,

avec le 1,3-diamino-2-propanol de formule (III)

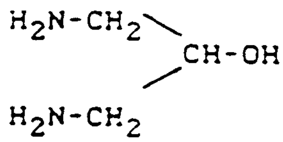

(III)

éventuellement en présence de diluants, à des températures entre 120 °C et 250 °C; ou bien

a3) lorsqu'on chauffe des sels d'acides carboxyliques du 1,3-diamino-2-propanol de formule (IIc)

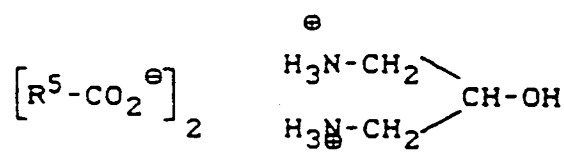

(IIc)

dans laquelle

$R^5$ a la signification indiquée ci-dessus, à des températures entre 120 °C et 250 °C,

et on isole éventuellement les sels de 5-hydroxy-3,4,5,6-tétrahydropyrimidinequi se forment répondant à la formule générale (IV)

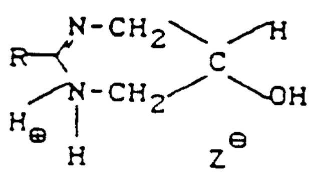

(IV)

dans laquelle

Z représente un atome de chlore ou $R^5$-$CO_2$ où $R^5$ a la signification indiquée ci-dessus et

R a la signification indiquée ci-dessus, et

b) on en libère éventuellement, à l'aide d'une base, les 5-hydroxy-3,4,5,6-tétrahydropyrimidines répondant à la formule générale (IVa)

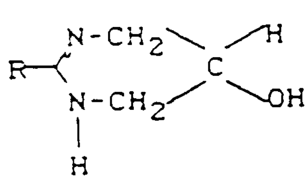

(IVa)

dans laquelle

R a la signification indiquée ci-dessus

et ensuite

c) on oxyde ou on soumet à une déshydrogénation, soit les sels de 5-hydroxy-3,4,5,6-tétrahydropyrimidine répondant à la formule générale (IV)

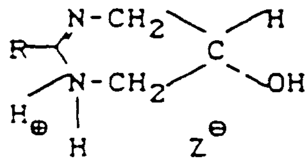

(IV)

dans laquelle

Z représente un atome de chlore ou $R^5$-$CO_2$ où $R^5$ a la signification indiquée ci-dessus et

R a la signification indiquée ci-dessus

soit les 5-hydroxy-3,4,5,6-tétrahydropyrimidines libres répondant à la formule générale (IVa)

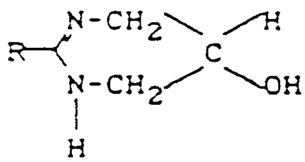

(IVa)

dans laquelle

R a la signification indiquée ci-dessus,

éventuellement après leur isolation pour obtenir les composés répondant à la formule générale (V)

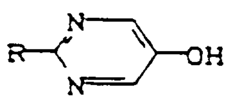

(V)

dans laquelle

R a la signification indiquée ci-dessus

et ensuite

d) on fait réagir les composés de formule (V) éventuellement après leur isolation, avec des composés répondant à la formule générale (VI)

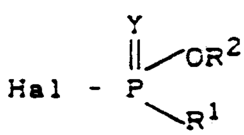

(VI)

dans laquelle

Hal représente un atome d'halogène et

Y, $R^1$ et $R^2$ ont la signification indiquée ci-dessus,

éventuellement en présence d'un agent neutralisateur d'acides et éventuellevent en présence d'un solvant, et on isole les composés de formule générale (I).

**2.** Procédé selon la revendication 1, dans lequel, dans la formule (I)

R représente un atome d'hydrogène, un groupe alcoxy contenant de 1 à 6 atomes de carbone, un groupe mono- ou dialkylamino contenant chacun de 1 à 4 atomes de carbone dans la fraction alkyle, ou encore un groupe alkyle contenant de 1 à 6 atomes de carbone éventuellement substitué par un groupe méthoxy, par un groupe éthoxy, par un groupe méthylsulfonyle ou par un groupe éthylsulfonyle, un groupe cycloalkyle contenant de 3 à 6 atomes de carbone éventuellement substitué par un groupe méthyle ou par un groupe éthyle, et un groupe

phényle éventuellement substitué par un groupe méthyle, par un groupe éthyle, par un groupe méthoxy, par un groupe éthoxy, par un groupe méthylsulfonyle ou par un groupe éthylsulfonyle.

**3.** Procédé selon la revendication 1, dans lequel, dans la formule (I)
R représente un groupe méthyle, un groupe isopropyle ou un groupe tert.-butyle.

**4.** Procédé selon les revendications 1 à 3, dans lequel, dans la formule (I)
$R^1$ représente un groupe éthoxy
$R^2$ représente un groupe éthyle ou un groupe s-butyle,
et
Y représente un atome de soufre.

**5.** Procédé pour la préparation des sels de 5-hydroxy-3,4,5,6-tétrahydropyrimidine répondant à la formule générale (IV)

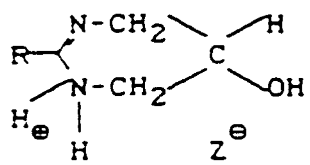

(IV)

ainsi que de leurs bases libres (formule IVa)
dans laquelle
Z et R ont la signification indiquée dans la revendication 1, à l'exception des composés dans lesquels R représente un atome d'hydrogène, un groupe méthyle ou un groupe chlorométhyle et Z représente un atome de chlore,
caractérisé en ce que
a1) on fait réagir des composés répondant à la formule générale (IIa)

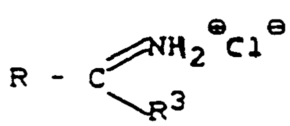

(IIa)

dans laquelle
R a la signification indiquée ci-dessus
$R^3$ représente un groupe amino ou le groupe $XR^4$ où
X représente un atome d'oxygène ou un atome de soufre et
$R^4$ représente un groupe méthyle ou un groupe éthyle,
avec le 1,3-diamino-2-propanol de formule (III)

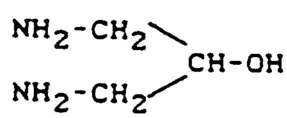

(III)

ou avec ses sels d'addition d'acides,
éventuellement en présence de diluants, à des températures entre 0 °C et 120 °C, ou bien
a2) on fait réagir des acides carboxyliques de formule (IIb)

$R^5-CO_2H$ (IIb)

dans laquelle
$R^5$ a la signification indiquée à la revendication 1,
avec le 1,3-diamino-2-propanol de formule (III)

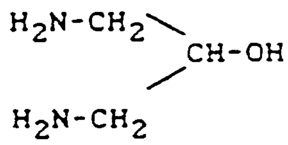

(III)

éventuellement en présence de diluants, à des températures entre 120 ° C et 250 ° C;
ou bien
a3) on chauffe des sels d'acides carboxyliques du 1,3-diamino-2-propanol de formule (IIc)

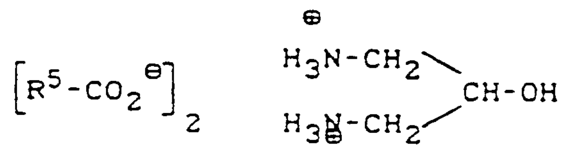

(IIc)

dans laquelle

$R^5$ a la signification indiquée dans la revendication 1, à des températures entre 120 ° C et 250 ° C,

et éventuellement, à partir des sels de 5-hydroxy-3,4,5,6-tétrahydropyrimidine obtenus, à l'aide d'une base, on libère les bases (formule IVa) de la manière habituelle.

**6.** Procédé pour la préparation des composés répondant à la formule générale (V)

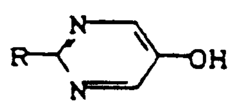

(V)

dans laquelle

R a la signification indiquée à la revendication 1, caractérisé en ce qu'on oxyde ou on soumet à une déshydrogénation les sels de 5-hydroxy-3,4,5,6-tétrahydropyrimidine répondant à la formule générale (IV)

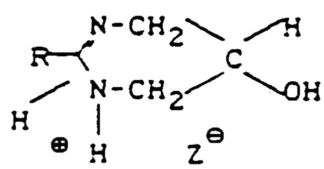

(IV)

dans laquelle

Z et R ont la signification indiquée à la revendication 1,

ou bien les 5-hydroxy-3,4,5,6-tétrahydropyrimidines libres répondant à la formule générale (IVa)

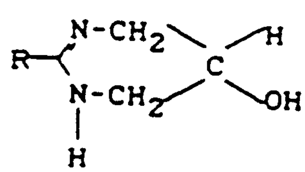

(IVa)

dans laquelle

R a la signification indiquée à la revendication 1,

éventuellement après leur isolation pour obtenir les composés répondant à la formule générale (V)

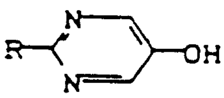

(V)

dans laquelle

R a la signification indiquée à la revendication 1.

**7.** Procédé selon la revendication 6, caractérisé en ce qu'on effectue l'oxydation ou la déshydrogénation en présence d'un solvant et d'une base, et éventuellement en présence d'un composé de métal de transition à des températures entre 0 °C et 300 °C, avec de l'oxygène ou des gaz contenant de l'oxygène.